# EUROPEAN PATENT SPECIFICATION

(11) **EP 3 335 696 B1**
(45) Date of publication and mention of the grant of the patent: **18.12.2019**
(21) Application number: 16397538.6
(22) Date of filing: 15.12.2016
(51) Int. Cl.: A61K 47/10, A61K 47/26, A61K 47/38, A61K 9/19, A61L 27/20, A61L 15/28, A61L 15/44, A61L 15/60, A61L 24/00, A61L 26/00, A61L 27/36, A61L 27/52, A61K 38/18, A61K 38/30, A61Q 19/00, A61K 8/98, A61K 35/35, A61L 27/54, A61L 27/56, A61K 8/60

(54) **A METHOD FOR DRYING CELL-FREE TISSUE EXTRACT IN A HYDROGEL COMPRISING NANOFIBRILLAR CELLULOSE AND A DRIED HYDROGEL COMPRISING NANOFIBRILLAR CELLULOSE AND CELL-FREE TISSUE EXTRACT**
VERFAHREN ZUM TROCKNEN VON ZELLFREIEN GEWEBEEXTRAKT IN EINEM HYDROGEL MIT NANOFIBRILLÄRER CELLULOSE UND GETROCKNETES HYDROGEL MIT NANOFIBRILLÄRER CELLULOSE UND ZELLFREIEM GEWEBEEXTRAKT
PROCÉDÉ DE SÉCHAGE D'UN EXTRAIT DE TISSU ACELLULAIRE DANS UN HYDROGEL COMPRENANT DE LA CELLULOSE NANOFIBRILLAIRE ET HYDROGEL DÉSHYDRATÉ COMPRENANT DE LA CELLULOSE NANOFIBRILLAIRE ET EXTRAIT DE TISSU ACELLULAIRE

(43) Date of publication of application: 20.06.2018
(73) Proprietor: UPM-Kymmene Corporation, 00100 Helsinki (FI); Everfill Oy, 00120 Helsinki (FI)
(72) Inventor: Luukko, Kari, 02660 Espoo (FI); Ylikomi, Timo, 38100 Karkku (FI); Sarkanen, Jertta-Riina, 36200 Kangasala (FI)
(74) Representative: Berggren Oy, Tampere

(56) References cited:
- EP-A1- 2 216 345
- WO-A1-2010/142850
- WO-A1-2013/072563
- US-A1- 2011 151 005
- MULLER A ET AL: "Bacterial nanocellulose with a shape-memory effect as potential drug delivery system", RSC ADVANCES: AN INTERNATIONAL JOURNAL TO FURTHER THE CHEMICAL SCIENCES, ROYAL SOCIETY OF CHEMISTRY, GB, vol. 4, no. 100, 1 January 2014 (2014-01-01), pages 57173-57184, XP008184804, ISSN: 2046-2069, DOI: 10.1039/C4RA09898F

## Description

### Technical field

The present disclosure relates to hydrogels comprising nanofibrillar cellulose comprising cell-free adipose tissue extract, which may be used as medical products in medical applications, and to methods for preparing and drying such hydrogels comprising cell-free adipose tissue extract. The medical uses include delivering bioactive agents to a target tissue.

### Background

Nanofibrillar cellulose refers to isolated cellulose fibrils or fibril bundles derived from cellulose raw material. Nanofibrillar cellulose is based on a natural polymer that is abundant in nature. Nanofibrillar cellulose has a capability of forming viscous hydrogel in water.

Nanofibrillar cellulose production techniques are based on grinding of aqueous dispersion of pulp fibers. The concentration of nanofibrillar cellulose in dispersions is typically very low, usually around 0.3-5%. After the grinding or homogenization process, the obtained nanofibrillar cellulose material is a dilute viscoelastic hydrogel.

Because of its nanoscale structure nanofibrillar cellulose has unique properties which enable functionalities which cannot be provided by conventional cellulose. However, for the same reason nanofibrillar cellulose is also a challenging material. For example dewatering or handling of nanofibrillar cellulose may be difficult. Further, after dewatering it is generally difficult to rehydrate or regel the dried material to obtain material having equal properties to the original nanofibrillar cellulose before the dewatering or drying. An especially challenging dewatering process is freeze-drying.

WO 2013/072563 A1 discloses drug delivery systems comprising a matrix comprising nanofibrillated cellulose.

US 2011/0151005 A1 discloses cell-free adipose tissue extracts for soft tissue engineering.

### Summary

The present disclosure provides a method for drying cell-free adipose tissue extract in a hydrogel comprising nanofibrillar cellulose, the method comprising
- providing a hydrogel comprising nanofibrillar cellulose,
- providing cell-free adipose tissue extract comprising a mixture of bioactive substances,
- providing polyethylene glycol,
- providing trehalose,
- mixing the hydrogel, the cell-free adipose tissue extract, the polyethylene glycol and the trehalose to obtain a mixture, and
- freeze drying the mixture to obtain a dried cell-free adipose tissue extract in a dried hydrogel comprising nanofibrillar cellulose.

Disclosed is a dried hydrogel comprising nanofibrillar cellulose, cell-free adipose tissue extract comprising a mixture of bioactive substances, polyethylene glycol and trehalose obtained with the method.

The present disclosure provides a dried hydrogel comprising nanofibrillar cellulose, cell-free adipose tissue extract comprising a mixture of bioactive substances, polyethylene glycol and trehalose, wherein the moisture content of the hydrogel is 10% (w/w) or less, such as in the range of 2-10% (w/w).

The main embodiments are characterized in the independent claims. Various embodiments are disclosed in the dependent claims. The embodiments recited in dependent claims and in the embodiments are mutually freely combinable unless otherwise explicitly stated.

It was surprisingly found out that when using both polyethylene glycol and trehalose in combination as cryoprotectants in the freeze drying process of cell-free tissue extract in a nanofibrillar cellulose hydrogel it was possible to obtain a dried product, which could be rehydrated or redispersed into a form which restores the original properties of the tissue extract in the hydrogel comprising nanofibrillar cellulose, *i.e*. the dried product can be regelled. Such properties include both the gel properties and the activity of the cell-free tissue extract, which could be further controllably released from the hydrogel. The presence of the selected cryoprotectants had no effect to the release profile of the bioactive agents from the gels. By drying the hydrogels it is possible to obtain a very long shelf life for medical products. Especially gels containing the active agents, which are unstable at moist conditions, such as proteins and agents sensitive to hydrolysis, can be successfully freeze-dried into forms containing little or practically no water and therefore having a prolonged stability and shelf life. Such freeze-dried medical products may be stored even at room temperature and may be regelled prior to use by adding liquid, such as water or saline. Further, the combination of polyethylene glycol and trehalose prevented precipitation of nanofibrillar cellulose in a solution or dispersion, which effect was not obtained by either of the cryoprotectants alone.

It was also found out that a hydrogel comprising nanofibrillar cellulose could acts as a material providing a controllable release of a wide variety of different molecules which could act for example as active therapeutic or cosmetic agents. Nanofibrillar cellulose hydrogel is able to provide a prolonged release of an active agent which effect can be applied to a variety of medical and cosmetic uses. The effect was obtained along a wide range of hydrogel concentrations and for a wide range of releasable molecules of different sizes and types.

Certain advantageous properties of the hydrogel comprising nanofibrillar cellulose include flexibility, elasticity and remouldability. As the hydrogel contains a lot of water, it may also show good permeability. These properties are useful for example when the hydrogel is used as a cover for healing wounds, or in other medical applications, such as for delivering therapeutic or cosmetic agents.

Flexibility is a feature which is desired in many applications, such as in medical applications. For example flexible patches and dressings comprising nanofibrillar cellulose hydrogel are useful for applying onto skin, for example for covering wounds and other damages or injuries, such as burns.

The hydrogels of the embodiments also provide high water retention capacity and molecule diffusion property speed, which properties are desired in medical applications such as wound healing and the like. Large hydrogels may be prepared and/or shaped which may be used for covering large areas.

The hydrogels described herein are useful in medical applications, wherein the materials comprising nanofibrillar cellulose are in contact with living tissue. It was discovered that nanofibrillar cellulose provides unusual properties when it is applied for example onto skin or onto a damaged area. The products containing nanofibrillar cellulose as described herein are highly biocompatible with the living tissue and provide several advantageous effects. Without binding to any specific theory, it is believed that a hydrogel comprising very hydrophilic nanofibrillar cellulose having a very high specific surface area, and thus high water retention ability, when applied against a skin or other tissue, provides favourable moist environment between the tissue or wound and the hydrogel comprising nanofibrillar cellulose. The high amount of free hydroxyl groups in the nanofibrillar cellulose forms hydrogen bonds between the nanofibrillar cellulose and water molecules and enables gel formation and the high water retention ability of the nanofibrillar cellulose. Because of the high amount of water in the nanofibrillar cellulose hydrogel, only water is supposed to be in contact with tissue, and which also enables migration of fluids and/or agents from the skin or wound to the hydrogel, or from the hydrogel to the skin or wound.

When the hydrogels are used for covering wounds or other damages or injuries, for example as such or as part of other products, such as plasters, dressings, medical patches or parts of plasters, patches or dressings, several effects are provided. The usability of the products is good as the product may be applied and removed easily without being damaged, for example torn. When used for covering wounds the hydrogel protects the wound from infection and keeps moist environment for the wound to heal.. The hydrogel will not attach to a damaged skin or wound in such irreversible way as conventional materials, which are usually very difficult to remove without damaging the healed area. The conditions between the product and the skin facilitate the healing of a damaged area.

The medical hydrogels of the embodiments are especially advantageous in the treatment of damaged skin, wherein the skin may be damaged at a variable depth or it may be even lost, or in treatment of grafts, such as skin grafts. The hydrogel may be used for covering the damaged or the graft area and it acts as a protective layer.

The hydrogels may also be used for controllably and effectively releasing and delivering bioactive agents, such as agents contained in cell-free tissue extracts, to a subject, such as a patient or a user, for example by transdermal route or by other route, such as directly to the damaged subcutaneous area. The controlled release refers for example to obtaining a desired release rate and/or profile of an agent or agents over a time period, which may be affected by the selection of the gel, for example the percentage of the gel or the thickness of the gel, the concentration or form of the releasable agent(s), presence of any auxiliary agents, or other conditions, such as pH, temperature and the like having an effect to the release rate and/or activity of the releasable agents. The combined effect of the special conditions between the tissue and the hydrogel as explained in previous and the release properties provides efficient delivery of substances into living tissue. The nanofibrillar cellulose hydrogel provides a hydrophilic matrix, which is non-toxic, biocompatible and also biodegradable. For example the matrix may be degraded enzymatically. On the other hand the hydrogel is stable at physiological conditions.

The cell free tissue extracts disclosed herein, especially the adipose tissue extract, may be used for example in soft tissue engineering. Soft tissue engineering seeks to fabricate replacement parts for soft tissue defects resulting from for example trauma, such as burns and scars, surgical resection or congenital malformations. In addition, cosmetic use, such as filling of facial wrinkles, is an important application of reconstituted soft tissue. Especially preferred target for treatment is the fat-containing subcutaneous soft tissue, also called as hypodermis, hypoderm, subcutis, or superficial fascia, which is the lowermost layer of the integumentary system in vertebrates. When a subcutaneous tissue is treated with the adipose tissue extract the bioactive substances of the extract facilitate the regeneration of the subcutaneous soft tissue and the skin.

### Detailed description

The present disclosure provides hydrogels comprising nanofibrillar cellulose, which may be also called as nanofibrillar cellulose hydrogels. The hydrogels may be provided as products, which may contain also other substances or other elements, such as reinforcing materials, covering materials, active agents, salts or the like. The hydrogels may be also provided or called as medical hydrogels or medical products.

More particularly the present application discloses a cell-free tissue extract comprising a mixture of bioactive substances in a hydrogel comprising nanofibrillar cellulose, polyethylene glycol and trehalose.

The present application discloses a medical hydrogel comprising nanofibrillar cellulose and cell-free tissue extract comprising a mixture of bioactive substances. Such hydrogels may be used for administering the bioactive substances to a subject, such as a patient, with or without the cryoprotectants described herein.

The term "medical" refers to a product or use wherein the product, i.e. a products comprising the hydrogel of the embodiments, is used or is suitable for medical purposes. A medical product may be sterilized, or it is sterilisable, for example by using temperature, pressure, moisture, chemicals, radiation or a combination thereof. The product may be for example autoclaved, or other methods using high temperature may be used, in which cases the product should tolerate high temperatures over 100°C, for example at least 121°C or 134°C. In one example the product is autoclaved at 121°C for 15 minutes. Such temperature treatments should be preferably carried out before adding any bioactive agents to the product to avoid denaturation of the bioactive agents. It is also desired that a medical product is pyrogen free and it does not contain undesired protein residues or the like. A medical product is preferably non-toxic to the target. Also UV sterilization may be used. A medical product may also be suitable for example for cosmetic purposes.

The nanofibrillar cellulose (NFC) hydrogel of the embodiments, such as anionic NFC hydrogel, is able to controllably release active agents as a function of time, especially when the temperature and pH are constant. It was found out that NFC hydrogel can be freeze-dried with the specific excipients and still be re-gelled. It was experimentally confirmed that the lyophilization of NFC hydrogel has practically no effect on the release profiles between a non-freeze-dried and a freeze-dried and re-gelled sample, that are otherwise identical. The results demonstrate that the freeze-drying method with PEG and trehalose together as cryoprotectants has practically no effect on the release capabilities of the used NFC hydrogels, such as anionic hydrogels. Anionic hydrogels are preferred for many applications. For example anionically modified nanofibrillar cellulose does not precipitate easily unlike the other grades. The aniconic grade is also especially suitable for releasing certain active agents.

In this specification, percentage values, unless specifically indicated otherwise, are based on weight (w/w). If any numerical ranges are provided, the ranges include also the upper and lower values.

Disclosed is a freeze-dryable cell-free tissue extract comprising a mixture of bioactive substances in a hydrogel comprising nanofibrillar cellulose, polyethylene glycol and trehalose. The polyethylene glycol and trehalose act as cryoprotecting agents providing a synergistic effect, which allows the nanofibrillar hydrogel to be freeze-dried in such way that a freeze-dried product is obtained having substantially identical properties to the hydrogel before the freeze-drying. When polyethylene glycol or trehalose alone were used, they showed no such remarkable cryoprotective effect which could indicate that they would be effective as cryoprotectants for nanofibrillar cellulose hydrogels, or for example prevent precipitation of the nanofibrillar cellulose. In one example the freeze-dryable hydrogel contains 0.1-2% (w/w) of polyethylene glycol and 0.05-1.0% (w/w) of trehalose calculated from the total mass of the hydrogel. The cell-free tissue extract is adipose tissue extract, such as human adipose tissue extract.

One embodiment provides a method for drying cell-free adipose tissue extract in a hydrogel comprising nanofibrillar cellulose, the method comprising
- providing a hydrogel comprising nanofibrillar cellulose,
- providing cell-free adipose tissue extract comprising a mixture of bioactive substances,
- providing polyethylene glycol,
- providing trehalose,
- mixing the hydrogel, the cell-free adipose tissue extract, the polyethylene glycol and the trehalose to obtain a mixture, and
- freeze drying the mixture to obtain the cell-free adipose tissue extract in the dried hydrogel comprising nanofibrillar cellulose.

Disclosed is a medical hydrogel comprising nanofibrillar cellulose and cell-free adipose tissue extract comprising a mixture of bioactive substances. Disclosed is a medical hydrogel comprising nanofibrillar cellulose and cell-free tissue extract comprising a mixture of bioactive substances and further one or more therapeutic or cosmetic agent(s). The medical hydrogel may be used for administering the substances or agent(s) to a subject. The medical hydrogel may also contain other ingredients, such as polyethylene glycol and trehalose, as described herein. The subject may be a patient or any other subject in need of the agents(s), such as human or animal.

### Cell-free tissue extracts

A tissue extract is combined with a hydrogel comprising nanofibrillar cellulose. The tissue extract is cell-free, or acellular, which terms may be used interchangeably. The cell-free tissue extract comprises a mixture of bioactive substances, which means that the tissue extract comprises more than one type of bioactive substance, which may be also called as bioactive agent. A bioactive substance may be a protein, a hormone, a factor, such as a growth factor, differentiation factor, or any other bioactive factor, or any other bioactive substance or a molecule. The bioactive substances may also include extracellular components, *i.e*. components present in the extracellular space, such as proteins and non-protein substances, for example DNA, RNA, or lipids, or extracellular fibrils such as collagens, elastin, proteoglycans, or extracellular vesicles, such as microvesicles or other vesicles, which may contain bioactive proteins or nucleic acids. The bioactive substances are obtained from the source cells, which may be originated from tissue or from cell culture. A bioactive substance, or bioactive agent or compound, is a substance that has an effect on a living organism, tissue or cell. The cell-free tissue extract does not refer to separately isolated proteins or other molecules, but rather to a group of substances obtained together from the source cells, which may be of one type or of more than one type. The cell-free tissue extract comprising a mixture of bioactive substances may be also herein called as cell-free tissue extract.

The claimed method and the claimed hydrogels are limited to those comprising cell-free extract from adipose tissue. Other tissues are disclosed in the following paragraphs for reference. Tissue as disclosed herein refers to any animal or plant tissue. In one example the tissue is animal tissue, such as human tissue. The tissue may be soft tissue, which includes the tissues that connect, support, or surround other structures and organs of the body. In one example the tissue does not include hard tissue such as bone. Examples of soft tissue include includes tendons, ligaments, fascia, skin, fibrous tissues, adipose tissue, and synovial membranes (which are connective tissue), and muscles, nerves and blood vessels (which are not connective tissue). The tissue as used herein also includes blood and other cell-containing fluids, such as body fluids and cell culture medium.

The tissue may be obtained from a body of an animal or a human. The cell-free tissue extract may be obtained from small tissue pieces or viable cells. In one embodiment the cell-free tissue extract is obtained from tissue, such as viable i.e. living cells, obtained directly from a body, for example a sample or tissue isolate obtained from a body, *i.e*. the cells are not cultured. The small tissue pieces refer to tissue obtained from a source, such as a body, and which has been cut in pieces but may not have been homogenized. A cell-free tissue extract is a solution obtained from source cells, for example a solution obtained by rupturing cells and removing all particulate matter, or a solution obtained by incubating viable cells in a solution without rupturing or homogenizing the cells and recovering the extract from the solution. The solution may be an aqueous solution, such as saline solution, buffer solution, or cell culture medium. When the cells are not ruptured or homogenized, only secreted and extracellular substances are extracted thus resulting in more limited amount of different substances in the extract. In one example the cell-free tissue extract, especially adipose tissue extract, is obtained from a body by liposuction. Tissue obtained by liposuction is a specific example of a homogenate-type of tissue isolate, wherein the cells are however not substantially ruptured or disintegrated, but at least part, preferably most, of the cells are viable. Cells obtained from removed tissue, such as biopsy, liposuction or the like, may contain extracellular components.

Disclosed in an extract obtained from cultured cells, such as cell cultured in a conditioned medium. A conditioned medium or media is a medium which has already been partially used by cells, for example a medium wherein the cells have been cultured for hours or for days. Although depleted of some components, it is enriched with cell derived material, such as small amounts of cytokines, growth factors and the like. Such cell conditioned medium will support the growth of cells at much lower density and may be mixed with some fresh medium. In such case the cell free tissue extract may be conditioned medium, or it may be derived from conditioned medium.

In general the extract may be filtered after being recovered from the cells. This may be carried out by using a filter having a such cut-off value which enables the solution with the bioactive substances to pass but prevent cells or cell debris from passing the filter. Another option to ensure that the cells are not included in the extract is to centrifuge the extract and recover the supernatant.

The source cells may be prokaryotic or eukaryotic cells. For example microbial cells may be included, such as bacterial cells or yeast cells. Eukaryotic cells may be plant cells or animal cells. Preferably the cells are animal cells, more particularly human cells. Examples of cells include stem cells, undifferentiated cells, precursor cells, as well as fully differentiated cells and combinations thereof. Examples of eukaryotic cells include transplantable cells, such as stem cells, for example omnipotent, pluripotent, multipotent, oligopotent or unipotent cells. In case of human embryonic stem cells the cells may be from a deposited cell line or made from unfertilized eggs, *i.e*. "parthenote" eggs or from parthenogenetically activated ovum, so that no human embryos are destroyed. In some examples the cells comprise cell types selected from the group consisting of keratocytes, keratinocytes, fibroblast cells, epithelial cells and combinations thereof. In some examples the cells are selected from the group consisting of stem cells, progenitor cells, precursor cells, connective tissue cells, epithelial cells, muscle cells, neuronal cells, endothelial cells, fibroblasts, keratinocytes, smooth muscle cells, stromal cells, mesenchymal cells, immune system cells, hematopoietic cells, dendritic cells, hair follicle cells and combinations thereof.

The cell-free tissue extract is not obtained from cultured cells. The cultured cells exhibit a different source cell type than cells obtained from a tissue obtained directly from a body, and they may have different end uses.

The cell-free tissue extract is adipose tissue extract, such as human adipose tissue extract. The products containing adipose tissue extract may be used to provide a microenvironment that is suitable for cell proliferation and differentiation, thus resulting in formation of adipose tissue without exogenous transplantation of cells. An optimal microenvironment enhances migration of adipose stem cells from the surrounding tissue and induces the cells to differentiate into mature adipocytes. Neovascularization in the developing tissue is required to avoid necrosis, scar formation and resorption of transplanted tissue, as well as to secrete several differentiation factors.

One embodiment provides a cell-free adipose tissue extract comprising at least VEGF, FGF-2, and IGF-1, preferably in predetermined amounts. The cell-free adipose tissue extract may further contain one or more of the other bioactive substances or other substances disclosed herein. In one example, the extract comprises at least 1 pg of VEGF, at least 70 pg of FGF-2, and at least 50 pg of IGF-1 per 1 mg of total protein. In one example, the VEGF content per 1 mg of total protein is at least 7 pg. The extract may be used in soft tissue repair or engineering and in angiogenesis induction in tissue engineering, such as in wound healing, in treating burn injuries and in treating ischemic conditions. One specific example of such use is the treatment of subcutaneous soft tissue loss.

One example provides a method for preparing the cell-free tissue extract, such as cell-free adipose tissue extract. The method comprises the steps of a) providing a non-homogenized tissue sample comprising viable cells, such as adipose tissue sample, b) incubating said sample a predetermined time in a solution, and c) collecting or recovering the extract from the solution, or collecting or recovering the solution as the extract. The method may be also include first washing or otherwise removing ruptured or homogenized cells to provide the sample comprising only or mostly viable cells. The method may also include finally filtering the extract to remove any remaining cells from the extract. Further example provides the cell-free tissue extract, such as a cell-free adipose tissue extract, obtainable with said method. The predetermined time may be for example in the range of 5 minutes to 48 hours, such as 1-24 hours, 0.5-2 hours, 1-2 hours, 0.5-6 hours, 0.5-12 hours, 1-12 hours, 2-12 hours, or 12-24 hours, for example about 1 hours, about 2 hours, about 24 hours or about 48 hours.

Adipose tissue comprises adipocytes of various differentiation states, endothelial cells, fibroblasts, pericytes as well as adipose stem cells and mesenchymal stem cells that are capable of differentiating into several lineages of cells. *De novo* adipogenesis, *i.e*. formation of new adipose tissue, is a promising approach to soft tissue engineering. The use of adipose tissue is based on studies attempting to provide a microenvironment that is suitable for cell proliferation and differentiation, thus resulting in formation of blood vessels and adipose tissue without exogenous transplantation of cells. An optimal microenvironment enhances migration of adipose stem cells from the surrounding tissue and induces differentiation of endothelial and adipocyte precursor cells into mature adipocytes, loose connective tissue and muscle cells and vascular cells. Neovascularization in the developing tissue is always required to avoid necrosis and scar formation, *i.e*. in order to enable the functional mature adipose tissue to develop.

The cell-free adipose tissue extract (ATE) described herein has potential for inducing rapid formation of adipose tissue and vasculature at the site of administration without administration of cells. The ATE is capable of creating an optimal microenvironment for *de novo* adipogenesis and angiogenesis and may thus be used in soft tissue repair and/or engineering.

The term "adipose tissue extract" (ATE) as used herein refers to a mixture of bioactive substances, preferably adipogenic and angiogenic factors, secreted by the adipose tissue cells, *i.e*. adipocytes of various differentiation states, endothelial cells, fibroblasts, pericytes as well as adipose stem cells. The adipose tissue extract differs from a traditional conditioned medium, for instance, in that the extract is collected from small tissue pieces or viable cells.

The preparation process of ATE includes no cell culturing, the resulting extract is much more concentrated than a conditioned medium, and the incubation time is short varying from several minutes to a couple of days.

Adipose tissue extract may be prepared from fat or adipose tissue sample obtained for example from liposuction or a surgical operation. If necessary, the tissue sample is cut into small pieces such that the cells remain substantially viable. Liposuction material may be used directly. In other words, processing of the tissue sample does not involve homogenization. The bioactive factors are extracted by incubating cells in a culture medium, in a sterile salt solution, in a phosphate buffered saline or in other suitable isotonic aqueous buffer solutions where cells or tissue pieces release bioactive factors into the liquid phase during incubation. The ATE, *i.e*. liquid phase without cells is then collected. The ATE may also be filtered prior to use to sterilize the extract and to ensure that the extract is a cell-free liquid. The resulting ATE is a cell-free mixture comprising proteins, such as cytokines, and other bioactive substances.

Not only autologous, but also allogenic adipose tissue may be used as a source of an ATE, and processed to that end as described above. Since the extract is acellular, immune responses and allergic reactions are unlikely even in allogenic use where the same extract is used for several patients. This is supported by animal studies conducted previously, where human ATE implanted in rats (xenograft) did not cause any inflammation reactions or other complications.

ATE is an optimal cytokine mixture of adipogenic and angiogenic factors that are expressed by the cells of adipose tissue. Bioactive substances of ATE may be measured by routine methods known in the art (for example ELISA). In a previous study the expression of 120 cytokines in various ATEs was measured. Long incubation time, such as 24 hours or more, resulted in a more angiogenic extract, whereas both short and long incubation times produced an adipogenic mixture.

An ATE may be tailored to comprise desired, or predetermined, amounts of adipogenic and angiogenic factors, including a vascular endothelial cell growth factor (VEGF), basic fibroblast growth factor (FGF-2), and insulin-like growth factor (IGF-1). The desired composition may depend on the intended use. According to the current knowledge, VEGF and FGF-2 are regarded as important factors for stimulating angiogenesis, whereas FGF-2 and IGF are important for stimulating adipogenesis. Thus, in one example the ATE comprises at least 1 pg of VEGF, at least 70 pg of FGF-2, and at least 50 pg of IGF-1 per 1 mg of total protein. Such an ATE is particularly suitable for stimulating adipogenesis for example in soft tissue repair and engineering. In another example, the ATE comprises at least 7 pg of VEGF, at least 70 pg of FGF-2, and at least 50 pg of IGF-1 per 1 mg of total protein. This kind of an ATE is particularly useful in stimulating angiogenesis for example in inducing wound healing and treating ischemic conditions and burn injuries. Different tailored ATEs may further contain different amounts of several other cytokines although they may not always be crucial for the specific application. In general, ATE should contain about 2:1 to about 1:2 IGF-1 and FGF-2, and about 1:100 to about 1:10 VEGF and FGF-2.

The content of an adipose tissue extract may be tailored by using different incubation times and temperatures in the preparation of the ATE. Typically, the incubation time ranges from a few minutes to several hours, such as overnight. For instance, an incubation time of about 1 hour typically results in a total protein content in the range of 1.7-2.5 mg/ml, IGF-1 content in the range of 200-1300 pg/ml, FGF-2 content in the range of 200-1400 pg/ml, and VEGF content in the range of 2-25 pg/ml. In other words, incubation time of 1 hour results typically in IGF-1 content in the range of 150-550 pg per 1 mg of total protein, in FGF-2 content in the range of 100-500 pg per 1 mg of total protein, and in VEGF content in the range of 3-20 pg per 1 mg of total protein. This type of extract is both adipogenic and angiogenic. If, however, an especially high concentration of VEGF (200-1400 pg/ml, that is 25-500 pg of VEGF per 1 mg of protein) is desirable in the extract, for example especially for angiogenesis induction in the desired target tissue, the incubation time should be extended for example to about 24 hours. Incubation time of 24 hour results typically in IGF-1 content in the range of 60-200 pg per 1 mg of total protein, in FGF-2 content in the range of 130-500 pg per 1 mg of total protein, and in VEGF content in the range of 25-500 pg per 1 mg of total protein. Furthermore, various incubation temperatures, typically ranging from room temperature to about 37°C, may be used to modify the adipose tissue extract. In some examples, however, lower temperatures such as 4°C may be used. In still other examples higher temperatures may be used as long as the proteins will not be denatured.

More generally, the concentration of VEGF in the extract may be in the range of 1-1400 pg/ml, and preferably in the range of 10-1000 pg/ml, more preferably in the range of 7-700 pg/ml, and most preferably in the range of 10-100 pg/ml. The concentration of FGF-2 in the extract may be in the range of 20-1500 pg/ml, preferably in the range of 100-1300 pg/ml, more preferably in the range of 150-1000 pg/ml, and most preferably in the range of 200-800 pg/ml. The concentration of IGF-1 may be in the range of 100-1500 pg/ml, and preferably in the range of 150-800 pg/ml, and most preferably in the range of 200-500 pg/ml. On the other hand, the total protein concentration of the extract may be in the range of 0.75-3.5 mg/ml. Preferably the total protein concentration is in the range of 1.4-2.7mg/ml, more preferably in the range of 1.8-2.7 mg/ml, and even more preferably in the range of 2-2.7 mg/ml, and most preferably in the range of 2.1-2.5 mg/ml.

The concentrations of the bioactive substances in the extract may also be defined in relation to the total protein concentration of the extract. Accordingly, the amount of VEGF in an extract containing 1 mg of total protein (growth factor content per 1 mg of protein) may be in the range of 1-800 pg, and preferably in the range of 3.5-500 pg, more preferably in the range of 7-300 pg, and most preferably in the range of 20-100 pg. The amount of FGF-2 in an extract containing about 1 mg of total protein (growth factor content per 1 mg of protein) may be in the range of 1-1200 pg, preferably in the range of 1-600 pg, more preferably in the range of 70-500 pg, and even more preferably in the range of 110-450 pg, and most preferably in the range of 250-350 pg. The amount of IGF-1 (growth factor content per 1 mg of protein) may be in the range of 50-700 pg, and preferably in the range of 100-500 pg, and even more preferably in the range of 100-300 pg, and most preferably in the range of 110-230 pg.

An ATE may further comprise several other bioactive substances or other substances, such as adipogenic and angiogenic factors, such as growth factors, interleukins, complement system products, glucocorticoids, prostaglandins, lipoproteins and free fatty acids as well as extracellular matrix components for example collagen I, collagen IV and collagen VI, proteoglycans, elastin, vesicles such as microvesicles containing regulatory proteins, peptides and/or nucleic acids, and hyaluronan either naturally or as added thereto to further tailor the ATE. In addition, the ATE may be supplemented, for instance, with any desired additional drugs or medicaments prior to use. Examples of substances which may be present in the ATE include angiogenin, adiponectin, TIMP-1 and TIMP-2, MIF, IGFBP-6, NAP-2, leptin, PDGF-BB, GRO, IL-6, MCP-1, leptin, CCL5 and VEGF.

On the other hand, an ATE may be further tailored by removing substances, such as single growth factors or cytokines, prior to use. For example, inhibitors of angiogenesis, such as TIMP-1 and TIMP-2, and primarily adipogenic factors such as IGF-1, adiponectin and/or leptin may be removed if the ATE is to be used for inducing primarily angiogenesis e.g. in treating ischemic conditions. Methods of removing or isolating desired substances are readily available in the art, including isolation of growth factors by immunoabsorption.

Tailoring an ATE may further include concentrating (for example by centrifugation) or diluting (for example with a culture medium, sterile physiological salt solution or any other buffered isotonic solution) in order to achieve a suitable ATE for a particular use.

In some examples, the ATE included in the hydrogel as described herein may be used as a cell culture medium or cell culture medium supplement to study adipogenesis or angiogenesis *in vitro.* The ATE results in even distribution of differentiated adipose cells in a cell culture and, thus, provides an excellent cell model for adipogenesis reflecting *in vivo* conditions in humans. To date, no adequate cell model for adipogenic differentiation exists. One example provides a method for inducing adipogenesis and a method of inducing angiogenesis. The methods comprise providing to a subject in need thereof the hydrogel or a medical product as described herein.

There is a general objective to replace xenogenic serum in cell culturing, especially in clinical applications. Human serum is extremely expensive and thus not an optimal solution. In some examples, an ATE may be used as a cell culture component or as a serum substitute in a cell culture medium. This is not only cost-effective but useful for generating tissue engineered human constructs, especially when animal derived components are not suitable.

The tissue extract, such as the adipose tissue extract, content in the hydrogel may vary. The tissue extract content may be expressed as protein content even if it may also contain some non-proteinous substances. Typically, the hydrogel may comprise protein in the range of 300 µg/ml - 1.2 mg/ml, preferably in the range of 600 µg/ml - 1 mg/ml, and even more preferably in the range of 800-900 µg/ml.

When preparing the hydrogels containing the cell-free extracts, the main starting material comprises nanofibrillar cellulose, which comprises or consists of cellulose fibrils having diameter in the submicron range. It forms a self-assembled hydrogel network even at low concentrations. These gels of nanofibrillar cellulose are highly shear thinning and pseudoplastic in nature.

### Nanofibrillar cellulose

The nanofibrillar cellulose is prepared normally from cellulose raw material of plant origin. The raw material may be based on any plant material that contains cellulose. The raw material may also be derived from certain bacterial fermentation processes. In one example the plant material is wood. Wood may be from softwood tree such as spruce, pine, fir, larch, douglas-fir or hemlock, or from hardwood tree such as birch, aspen, poplar, alder, eucalyptus, oak, beech or acacia, or from a mixture of softwoods and hardwoods. In one example the nanofibrillar cellulose is obtained from wood pulp. In one example the nanofibrillar cellulose is obtained from hardwood pulp. In one example the hardwood is birch. In one example the nanofibrillar cellulose is obtained from softwood pulp.

The nanofibrillar cellulose is preferably made of plant material. In one example the fibrils are obtained from non-parenchymal plant material. In such case the fibrils may be obtained from secondary cell walls. One abundant source of such cellulose fibrils is wood fibres. The nanofibrillar cellulose is manufactured by homogenizing wood-derived fibrous raw material, which may be chemical pulp. Cellulose fibers are disintegrated to produce fibrils which have the diameter of only some nanometers, which is 50 nm at the most, for example in the range of 1-50 µm, and gives a dispersion of fibrils in water. The fibrils may be reduced to size where the diameter of most of the fibrils is in the range of only 2-20 nm. The fibrils originating from secondary cell walls are essentially crystalline with degree of crystallinity of at least 55%. Such fibrils may have different properties than fibrils originated from primary cell walls, for example the dewatering of fibrils originating from secondary cell walls may be more challenging.

As used herein, the term "nanofibrillar cellulose" refers to cellulose fibrils or fibril bundles separated from cellulose-based fiber raw material. These fibrils are characterized by a high aspect ratio (length/diameter): their length may exceed 1 µm, whereas the diameter typically remains smaller than 200 nm. The smallest fibrils are in the scale of so-called elementary fibrils, the diameter being typically in the range of 2-12 nm. The dimensions and size distribution of the fibrils depend on the refining method and efficiency. Nanofibrillar cellulose may be characterized as a cellulose-based material, in which the median length of particles (fibrils or fibril bundles) is not greater than 50 µm, for example in the range of 1-50 µm, and the particle diameter is smaller than 1 µm, suitably in the range of 2-500 nm. In case of native nanofibrillar cellulose, in one example the average diameter of a fibril is in the range of 5-100 nm, for example in the range of 10-50 nm. Nanofibrillar cellulose is characterized by a large specific surface area and a strong ability to form hydrogen bonds. In water dispersion, the nanofibrillar cellulose typically appears as either light or turbid gel-like material. Depending on the fiber raw material, nanofibrillar cellulose may also contain small amounts of other wood components, such as hemicellulose or lignin. The amount is dependent on the plant source. Often used parallel names for nanofibrillar cellulose include nanofibrillated cellulose (NFC) and nanocellulose.

Different grades of nanofibrillar cellulose may be categorized based on three main properties: (i) size distribution, length and diameter (ii) chemical composition, and (iii) rheological properties. To fully describe a grade, the properties may be used in parallel. Examples of different grades include native (or non-modified) NFC, oxidized NFC (high viscosity), oxidized NFC (low viscosity), carboxymethylated NFC and cationized NFC. Within these main grades, also sub-grades exist, for example: extremely well fibrillated vs. moderately fibrillated, high degree of substitution vs. low, low viscosity vs. high viscosity etc. The fibrillation technique and the chemical pre-modification have an influence on the fibril size distribution. Typically, non-ionic grades have wider fibril diameter (for example in the range of 10-100 nm, or 10-50 nm) while the chemically modified grades are a lot thinner (for example in the range of 2-20 nm). Distribution is also narrower for the modified grades. Certain modifications, especially TEMPO-oxidation, yield shorter fibrils.

Depending on the raw material source, e.g. hardwood (HW) vs. softwood (SW) pulp, different polysaccharide composition exists in the final nanofibrillar cellulose product. Commonly, the non-ionic grades are prepared from bleached birch pulp, which yields high xylene content (25% by weight). Modified grades are prepared either from HW or SW pulps. In those modified grades, the hemicelluloses are also modified together with the cellulose domain. Most probably, the modification is not homogeneous, i.e. some parts are more modified than others. Thus, detailed chemical analysis is not possible - the modified products are always complicated mixtures of different polysaccharide structures.

In an aqueous environment, a dispersion of cellulose nanofibers forms a viscoelastic hydrogel network. The gel is formed at relatively low concentrations of, for example, 0.05-0.2% (w/w) by dispersed and hydrated entangled fibrils. The viscoelasticity of the NFC hydrogel may be characterized, for example, with dynamic oscillatory rheological measurements.

The nanofibrillar cellulose hydrogels exhibit characteristic rheological properties. For example they are shear-thinning or pseudoplastic materials, which means that their viscosity depends on the speed (or force) by which the material is deformed. When measuring the viscosity in a rotational rheometer, the shear-thinning behavior is seen as a decrease in viscosity with increasing shear rate. The hydrogels show plastic behavior, which means that a certain shear stress (force) is required before the material starts to flow readily. This critical shear stress is often called the yield stress. The yield stress can be determined from a steady state flow curve measured with a stress controlled rheometer. When the viscosity is plotted as function of applied shear stress, a dramatic decrease in viscosity is seen after exceeding the critical shear stress. The zero shear viscosity and the yield stress are the most important rheological parameters to describe the suspending power of the materials. These two parameters separate the different grades quite clearly and thus enable classification of the grades.

The dimensions of the fibrils or fibril bundles are dependent on the raw material and the disintegration method. Mechanical disintegration of the cellulose raw material may be carried out with any suitable equipment such as a refiner, grinder, disperser, homogenizer, colloider, friction grinder, pin mill, rotor-rotor dispergator, ultrasound sonicator, fluidizer such as microfluidizer, macrofluidizer or fluidizer-type homogenizer. The disintegration treatment is performed at conditions wherein water is sufficiently present to prevent the formation of bonds between the fibers.

In one example the disintegration is carried out by using a disperser having at least one rotor, blade or similar moving mechanical member, such as a rotor-rotor dispergator, which has at least two rotors. In a disperser the fiber material in dispersion is repeatedly impacted by blades or ribs of rotors striking it from opposite directions when the blades rotate at the rotating speed and at the peripheral speed determined by the radius (distance to the rotation axis) in opposite directions. Because the fiber material is transferred outwards in the radial direction, it crashes onto the wide surfaces of the blades, *i.e*. ribs, coming one after the other at a high peripheral speed from opposite directions; in other words, it receives several successive impacts from opposite directions. Also, at the edges of the wide surfaces of the blades, *i.e*. ribs, which edges form a blade gap with the opposite edge of the next rotor blade, shear forces occur, which contribute to the disintegration of the fibers and detachment of fibrils. The impact frequency is determined by the rotation speed of the rotors, the number of the rotors, the number of blades in each rotor, and the flow rate of the dispersion through the device.

In a rotor-rotor dispergator the fiber material is introduced through counter-rotating rotors, outwards in the radial direction with respect to the axis of rotation of the rotors in such a way that the material is repeatedly subjected to shear and impact forces by the effect of the different counter-rotating rotors, whereby it is simultaneously fibrillated. One example of a rotor-rotor dispergator is an Atrex device.

Another example of a device suitable for disintegrating is a pin mill, such as a multi-peripheral pin mill. One example of such device, as described in US 6202946 B1, includes a housing and in it a first rotor equipped with collision surfaces; a second rotor concentric with the first rotor and equipped with collision surfaces, the second rotor being arranged to rotate in a direction opposite to the first rotor; or a stator concentric with the first rotor and equipped with collision surfaces. The device includes a feed orifice in the housing and opening to the center of the rotors or the rotor and stator, and a discharge orifice on the housing wall and opening to the periphery of the outermost rotor or stator.

In one example the disintegrating is carried out by using a homogenizer. In a homogenizer the fiber material is subjected to homogenization by an effect of pressure. The homogenization of the fiber material dispersion to nanofibrillar cellulose is caused by forced through-flow of the dispersion, which disintegrates the material to fibrils. The fiber material dispersion is passed at a given pressure through a narrow through-flow gap where an increase in the linear velocity of the dispersion causes shearing and impact forces on the dispersion, resulting in the removal of fibrils from the fiber material. The fiber fragments are disintegrated into fibrils in the fibrillating step.

As used herein, the term "fibrillation" generally refers to disintegrating fiber material mechanically by work applied to the particles, where cellulose fibrils are detached from the fibers or fiber fragments. The work may be based on various effects, like grinding, crushing or shearing, or a combination of these, or another corresponding action that reduces the particle size. The energy taken by the refining work is normally expressed in terms of energy per processed raw material quantity, in units of e.g. kWh/kg, MWh/ton, or units proportional to these. The expressions "disintegration" or "disintegration treatment" may be used interchangeably with "fibrillation".

The fiber material dispersion that is subjected to fibrillation is a mixture of fiber material and water, also herein called "pulp". The fiber material dispersion may refer generally to whole fibers, parts (fragments) separated from them, fibril bundles, or fibrils mixed with water, and typically the aqueous fiber material dispersion is a mixture of such elements, in which the ratios between the components are dependent on the degree of processing or on the treatment stage, for example number of runs or "passes" through the treatment of the same batch of fiber material.

One way to characterize the nanofibrillar cellulose is to use the viscosity of an aqueous solution containing said nanofibrillar cellulose. The viscosity may be, for example, Brookfield viscosity or zero shear viscosity. The specific viscosity, as described herein, distinguishes nanofibrillar cellulose from non-nanofibrillar cellulose.

In one example the apparent viscosity of the nanofibrillar cellulose is measured with a Brookfield viscometer (Brookfield viscosity) or another corresponding apparatus. Suitably a vane spindle (number 73) is used. There are several commercial Brookfield viscometers available for measuring apparent viscosity, which all are based on the same principle. Suitably RVDV spring (Brookfield RVDV-III) is used in the apparatus. A sample of the nanofibrillar cellulose is diluted to a concentration of 0.8% by weight in water and mixed for 10 min. The diluted sample mass is added to a 250 ml beaker and the temperature is adjusted to 20°C±1 °C, heated if necessary and mixed. A low rotational speed 10 rpm is used.

The nanofibrillar cellulose provided as a starting material in the method may be characterized by the viscosity it provides in a water solution. The viscosity describes, for example, the fibrillation degree of the nanofibrillar cellulose. In one embodiment the nanofibrillar cellulose when dispersed in water provides a Brookfield viscosity of at least 2000 mPa·s, such as at least 3000 mPa·s, measured at a consistency of 0.8% (w/w) and at 10 rpm. In one embodiment the nanofibrillar cellulose, when dispersed in water, provides a Brookfield viscosity of at least 10000 mPa·s measured at a consistency of 0.8% (w/w) and at 10 rpm. In one example the nanofibrillar cellulose, when dispersed in water, provides a Brookfield viscosity of at least 15000 mPa·s measured at a consistency of 0.8% (w/w) and at 10 rpm. Examples of Brookfield viscosity ranges of said nanofibrillar cellulose when dispersed in water include 2000-20000 mPa·s, 3000-20000 mPa·s, 10000-20000 mPa·s, 15000-20000 mPa·s, 2000-25000 mPa·s, 3000-25000 mPa·s, 10000-25000 mPa·s, 15000-25000 mPa·s, 2000-30000 mPa·s, 3000-30000 mPa·s, 10000-30000 mPa·s, and 15000-30000 mPa·s, measured at a consistency of 0.8% (w/w) and at 10 rpm.

In one embodiment the nanofibrillar cellulose comprises non-modified nanofibrillar cellulose. In one embodiment the nanofibrillar cellulose is non-modified nanofibrillar cellulose. It was found out that the drainage of non-modified nanofibrillar cellulose was significantly faster than with for example anionic grade. Non-modified nanofibrillar cellulose generally has a Brookfield viscosity in the range of 2000-10000 mPa·s, measured at a consistency of 0.8% (w/w) and at 10 rpm.

The disintegrated fibrous cellulosic raw material may be modified fibrous raw material. Modified fibrous raw material means raw material where the fibers are affected by the treatment so that cellulose nanofibrils are more easily detachable from the fibers. The modification is usually performed to fibrous cellulosic raw material which exists as a suspension in a liquid, that is, pulp.

The modification treatment to the fibers may be chemical or physical. In chemical modification the chemical structure of cellulose molecule is changed by chemical reaction ("derivatization" of cellulose), preferably so that the length of the cellulose molecule is not affected but functional groups are added to β-D-glucopyranose units of the polymer. The chemical modification of cellulose takes place at a certain conversion degree, which is dependent on the dosage of reactants and the reaction conditions, and as a rule it is not complete so that the cellulose will stay in solid form as fibrils and does not dissolve in water. In physical modification anionic, cationic, or non-ionic substances or any combination of these are physically adsorbed on cellulose surface. The modification treatment may also be enzymatic.

The cellulose in the fibers may be especially ionically charged after the modification, because the ionic charge of the cellulose weakens the internal bonds of the fibers and will later facilitate the disintegration to nanofibrillar cellulose. The ionic charge may be achieved by chemical or physical modification of the cellulose. The fibers may have higher anionic or cationic charge after the modification compared with the starting raw material. Most commonly used chemical modification methods for making an anionic charge are oxidation, where hydroxyl groups are oxidized to aldehydes and carboxyl groups, sulphonization and carboxymethylation. A cationic charge in turn may be created chemically by cationization by attaching a cationic group to the cellulose, such as quaternary ammonium group.

In one embodiment the nanofibrillar cellulose comprises chemically modified nanofibrillar cellulose, such as anionically modified nanofibrillar cellulose or cationically modified nanofibrillar cellulose. In one embodiment the nanofibrillar cellulose is anionically modified nanofibrillar cellulose. In one embodiment the nanofibrillar cellulose is cationically modified nanofibrillar cellulose. In one example the anionically modified nanofibrillar cellulose is oxidized nanofibrillar cellulose. In one example the anionically modified nanofibrillar cellulose is sulphonized nanofibrillar cellulose. In one example the anionically modified nanofibrillar cellulose is carboxymethylated nanofibrillar cellulose. Chemically modified nanofibrillar celluloses may be used to affect to the release profile of certain active agents. For example anionic grades may be used to release cationically charged molecules to obtain a prolonged release rate, or vice versa.

The cellulose may be oxidized. In the oxidation of cellulose, the primary hydroxyl groups of cellulose may be oxidized catalytically by a heterocyclic nitroxyl compound, for example 2,2,6,6-tetramethylpiperidinyl-1-oxy free radical, generally called "TEMPO". The primary hydroxyl groups (C6-hydroxyl groups) of the cellulosic β-D-glucopyranose units are selectively oxidized to carboxylic groups. Some aldehyde groups are also formed from the primary hydroxyl groups. Regarding the finding that low degree of oxidation does not allow efficient enough fibrillation and higher degree of oxidation inflicts degradation of cellulose after mechanical disruptive treatment, the cellulose may be oxidized to a level having a carboxylic acid content in the oxidized cellulose in the range of 0.6-1.4 mmol COOH/ g pulp, or 0.8-1.2 mmol COOH / g pulp, preferably to 1.0-1.2 mmol COOH/ g pulp, determined by conductometric titration. When the fibers of oxidized cellulose so obtained are disintegrated in water, they give stable transparent dispersion of individualized cellulose fibrils, which may be, for example, of 3-5 nm in width. With oxidized pulp as the starting medium, it is possible to obtain nanofibrillar cellulose where Brookfield viscosity measured at a consistency of 0.8% (w/w) is at least 10000 mPa·s, for example in the range of 10000-30000 mPa·s.

Whenever the catalyst "TEMPO" is mentioned in this disclosure, it is evident that all measures and operations where "TEMPO" is involved apply equally and analogously to any derivative of TEMPO or any heterocyclic nitroxyl radical capable of catalyzing selectively the oxidation of the hydroxyl groups of C6 carbon in cellulose.

In one example such chemically modified nanofibrillar cellulose, when dispersed in water, provides a Brookfield viscosity of at least 10000 mPa·s measured at a consistency of 0.8% (w/w) and at 10 rpm. In one example such chemically modified nanofibrillar cellulose, when dispersed in water, provides a Brookfield viscosity of at least 15000 mPa·s measured at a consistency of 0.8% (w/w) and at 10 rpm. In one example such chemically modified nanofibrillar cellulose, when dispersed in water, provides a Brookfield viscosity of at least 18000 mPa·s measured at a consistency of 0.8% (w/w) and at 10 rpm. Examples of anionic nanofibrillar celluloses used have a Brookfield viscosity in the range of 13000-15000 mPa·s or 18000-20000 mPa·s, or even up to 25000 mPa·s, depending on the degree of fibrillation.

In one embodiment the nanofibrillar cellulose is TEMPO oxidized nanofibrillar cellulose. It provides high viscosity at low concentrations, for example a Brookfield viscosity of at least 20000 mPa·s, even at least 25000 mPa·s, measured at a consistency of 0.8% (w/w) and at 10 rpm. In one example the Brookfield viscosity of TEMPO oxidized nanofibrillar cellulose is in the range of 20000-30000 mPa·s, such as 25000-30000 mPa·s, measured at a consistency of 0.8% (w/w) and at 10 rpm.

In one embodiment the nanofibrillar cellulose comprises chemically unmodified nanofibrillar cellulose. In one example such chemically unmodified nanofibrillar cellulose, when dispersed in water, provides a Brookfield viscosity of at least 2000 mPa·s, or at least 3000 mPa·s, measured at a consistency of 0.8% (w/w) and at 10 rpm.

The nanofibrillar cellulose may also be characterized by the average diameter (or width), or by the average diameter together with the viscosity, such as Brookfield viscosity or zero shear viscosity. In one example said nanofibrillar cellulose has a number average diameter of a fibril in the range of 1-100 nm. In one example said nanofibrillar cellulose has a number average diameter of a fibril in the range of 1-50 nm. In one example said nanofibrillar cellulose has a number average diameter of a fibril in the range of 2-15 nm, such as TEMPO oxidized nanofibrillar cellulose.

The diameter of a fibril may be determined with several techniques, such as by microscopy. Fibril thickness and width distribution may be measured by image analysis of the images from a field emission scanning electron microscope (FE-SEM), a transmission electron microscope (TEM), such as a cryogenic transmission electron microscope (cryo-TEM), or an atomic force microscope (AFM). In general AFM and TEM suit best for nanofibrillar cellulose grades with narrow fibril diameter distribution.

In one example a rheometer viscosity of the nanofibrillar cellulose dispersion is measured at 22°C with a stress controlled rotational rheometer (AR-G2, TA Instruments, UK) equipped with a narrow gap vane geometry (diameter 28 mm, length 42 mm) in a cylindrical sample cup having a diameter of 30 mm. After loading the samples to the rheometer they are allowed to rest for 5 min before the measurement is started. The steady state viscosity is measured with a gradually increasing shear stress (proportional to applied torque) and the shear rate (proportional to angular velocity) is measured. The reported viscosity (=shear stress/shear rate) at a certain shear stress is recorded after reaching a constant shear rate or after a maximum time of 2 min. The measurement is stopped when a shear rate of 1000 s⁻¹ is exceeded. This method may be used for determining the zero-shear viscosity.

In one example the nanofibrillar cellulose, when dispersed in water, provides a zero shear viscosity ("plateau" of constant viscosity at small shearing stresses) in the range of 1000-100000 Pa·s, such as in the range of 5000-50000 Pa·s, and a yield stress (shear stress where the shear thinning begins) in the range of 1-50 Pa, such as in the range of 3-15 Pa, determined by rotational rheometer at a consistency of 0.5% (w/w) by weight in aqueous medium.

Turbidity is the cloudiness or haziness of a fluid caused by individual particles (total suspended or dissolved solids) that are generally invisible to the naked eye. There are several practical ways of measuring turbidity, the most direct being some measure of attenuation (that is, reduction in strength) of light as it passes through a sample column of water. The alternatively used Jackson Candle method (units: Jackson Turbidity Unit or JTU) is essentially the inverse measure of the length of a column of water needed to completely obscure a candle flame viewed through it.

Turbidity may be measured quantitatively using optical turbidity measuring instruments. There are several commercial turbidometers available for measuring turbidity quantitatively. In the present case the method based on nephelometry is used. The units of turbidity from a calibrated nephelometer are called Nephelometric Turbidity Units (NTU). The measuring apparatus (turbidometer) is calibrated and controlled with standard calibration samples, followed by measuring of the turbidity of the diluted NFC sample.

In one turbidity measurement method, a nanofibrillar cellulose sample is diluted in water, to a concentration below the gel point of said nanofibrillar cellulose, and turbidity of the diluted sample is measured. Said concentration where the turbidity of the nanofibrillar cellulose samples is measured is 0.1%. HACH P2100 Turbidometer with a 50 ml measuring vessel is used for turbidity measurements. The dry matter of the nanofibrillar cellulose sample is determined and 0.5 g of the sample, calculated as dry matter, is loaded in the measuring vessel, which is filled with tap water to 500 g and vigorously mixed by shaking for about 30 s. Without delay the aqueous mixture is divided into 5 measuring vessels, which are inserted in the turbidometer. Three measurements on each vessel are carried out. The mean value and standard deviation are calculated from the obtained results, and the final result is given as NTU units.

One way to characterize nanofibrillar cellulose is to define both the viscosity and the turbidity. Low turbidity refers to small size of the fibrils, such as small diameter, as small fibrils scatter light poorly. In general as the fibrillation degree increases, the viscosity increases and at the same time the turbidity decreases. This happens, however, until a certain point. When the fibrillation is further continued, the fibrils finally begin to break and cannot form a strong network any more. Therefore, after this point, both the turbidity and the viscosity begin to decrease.

In one example the turbidity of anionic nanofibrillar cellulose is lower than 90 NTU, for example from 3 to 90 NTU, such as from 5 to 60, for example 8-40 measured at a consistency of 0.1% (w/w) in aqueous medium, and measured by nephelometry. In one example the turbidity of native nanofibrillar may be even over 200 NTU, for example from 10 to 220 NTU, such as from 20 to 200, for example 50-200 measured at a consistency of 0.1% (w/w) in aqueous medium, and measured by nephelometry. To characterize the nanofibrillar cellulose these ranges may be combined with the viscosity ranges of the nanofibrillar cellulose, such as nanofibrillar cellulose which, when dispersed in water, provides a Brookfield viscosity of at least 2000 mPa·s, at least 3000 mPa·s, at least 5000 mPa·s, such as at least 10000 mPa·s, for example at least 15000 mPa·s measured at a consistency of 0.8% (w/w) and at 10 rpm.

The starting material for the preparation process is usually nanofibrillar cellulose obtained directly from the disintegration of some of the above-mentioned fibrous raw material and existing at a relatively low concentration homogeneously distributed in water due to the disintegration conditions. The starting material may be an aqueous gel at a concentration of 0.2-10%.

In one example the concentration of the nanofibrillar cellulose in the hydrogel before the freeze-drying is in the range of 0.5-10%, 1-10%, such as 2-8% or 1-7%. A preferable range useful for most applications is 3-7%, 4-7% or 4-8%. In the tests the concentrations 3% and 6.5% were selected to represent concentrations near the ends of these ranges. The same concentrations of the hydrogels may be restored after freeze-drying from the freeze-dried gels. More particularly dried gel is redispersable in water and will give, when redispersed in water, for example at a dispergation concentration in the range of 0.1-10% (w/w), such as in the range of 0.5-2.0 % (w/w) or 2-8% (w/w) or 3-7% (w/w), a viscosity profile that is equal or substantially equal to the viscosity profile it had originally at the same dispergation concentration.

### Polyethylene glycol

Polyethylene glycol (PEG) is a polyether compound also known as polyethylene oxide (PEO) or polyoxyethylene (POE), depending on its molecular weight. The structure of PEG is commonly expressed as H-(O-CH₂-CH₂)ₙ-OH. In general polyethylene glycols are prepared by polymerization of ethylene oxide and are commercially available over a wide range of molecular weights from 300 g/mol to 10000000 g/mol. Polyethylene glycol is water-soluble and it has a low toxicity.

In one example the polyethylene glycol has a molecular weight in the range of 100-10000 kDa, such as 1000-10000 kDa. In one example the polyethylene glycol has a molecular weight in the range of 3000-8000 kDa, such as 5000-7000 kDa, for example about 6000 kDa. A "molecular weight" as used in this disclosure may refer to number average molar mass. In general the number average molecular mass of a polymer can be determined for example by gel permeation chromatography, viscometry via the (Mark-Houwink equation), colligative methods such as vapor pressure osmometry, end-group determination or proton NMR.

### Trehalose

Trehalose, also known as α,α-trehalose; α-D-glucopyranosyl-(1→1)-α-D-glucopyranoside, mycose or tremalose, is a natural alpha-linked disaccharide formed by an α,α-1,1-glucoside bond between two α-glucose units. Trehalose is nutritionally equivalent to glucose, because it is rapidly broken down into glucose by the enzyme trehalase. Trehalose may be present as anhydrous or as dihydrate. In one example the trehalose is D(+)-trehalose dehydrate, which is compatible with nanofibrillar cellulose. In one example the trehalose is D-(+)-trehalose dehydrate. It may be provided as solid or as dissolved in an aqueous medium, such as water.

### Preparation of the freeze dried product

The method comprises providing the ingredients nanofibrillar cellulose, in general as an aqueous suspension or hydrogel, cell-free tissue extract, polyethylene glycol and trehalose. In one example the nanofibrillar cellulose is the only cellulosic material in the aqueous suspension or in the hydrogel. In one example the nanofibrillar cellulose is the only polymeric gel-forming material in the aqueous suspension or in the hydrogel. In one example the aqueous suspension or the hydrogel comprises an amount of another fibrous material, such as non-nanofibrillar cellulose, for example an amount of 0.1-2% (w/w) of the dry weight of the fibrous material.

The method comprises providing a hydrogel comprising nanofibrillar cellulose. The concentration of the nanofibrillar cellulose in the hydrogel may in the range of 0.1-10% (w/w), 0.5-10% (w/w), or 1-10% (w/w), such as 2-8% (w/w). A preferable range useful for most applications is 3-6.5% (w/w), 2.5-7% (w/w), 3-7% (w/w), 4-7% (w/w) or 4-8% (w/w).

The method comprises providing a cell-free tissue extract comprising a mixture of bioactive substances. The concentration of the cell-free tissue extract may be defined as the wet mass of the extract in the hydrogel or the mixture, as dry content of the cell-free tissue extract in the hydrogel or the mixture, or as dry protein content in the hydrogel or the mixture, preferably in the final hydrogel or in the mixture containing all the ingredients. The dry content of the cell-free tissue extract in the hydrogel or in the mixture may be in the range of 0.01-20% (w/w). The dry content of the cell-free tissue extract may be in the range of 0.01-10% (w/w), such as 0.05-10% (w/w) calculated from the total mass of the hydrogel or the mixture. In one example the dry content of the cell-free tissue extract in the hydrogel or in the mixture is in the range of 0.1-5% (w/w), such as 0.1-3% (w/w), 0.5-3.5% (w/w) or 0.5-5% (w/w) calculated from the total mass of the hydrogel or the mixture. In one example the dry content of the cell-free tissue extract refers to dry total protein content of the cell-free tissue extract.

The method further comprises mixing the hydrogel, the cell-free tissue extract, the polyethylene glycol and the trehalose to obtain a mixture. The ingredients may be mixed in any order to obtain an aqueous mixture containing nanofibrillar cellulose, cell-free tissue extract, polyethylene glycol and trehalose. In one embodiment the mixture contains 0.1-2% (w/w) of polyethylene glycol and/or 0.05-1.0% (w/w) of trehalose calculated from the total mass of the mixture. In one example the mixture contains 0.1-2% (w/w) of polyethylene glycol and/or 0.1-0.5% (w/w) of trehalose calculated from the total mass of the mixture. In one example the mixture contains 0.5-1.5% (w/w) of polyethylene glycol and/or 0.1-0.5% (w/w) of trehalose calculated from the total mass of the mixture. In one example the mixture contains 0.5-1.5% (w/w) of polyethylene glycol and/or 0.3-0.4% (w/w) of trehalose calculated from the total mass of the mixture. The mixture is in a form of a hydrogel and it may be also called as hydrogel, more particularly a hydrogel comprising nanofibrillar cellulose and preferably other ingredients, such as polyethylene glycol, trehalose and/or other ingredients such as cell-free tissue extract and any other possible active agents as described herein. In one example the trehalose is present in the range of 5-100 mmol, such as 10-50 mmol, in the mixture. The mixing may be carried out using any suitable mixer or homogenizer, or the ingredients may be mixed by connecting two syringes containing the mixture and injecting the mixture repeatedly between the syringes to obtain mixing and/or homogenization, as was done in the experiments carried out at laboratory scale.

The ratio of dry nanofibrillar cellulose in the gel to the cryoprotectants (NFC:PEG:trehalose by weight) may be for example about 9.5:3:1 to 20:3:1 which ranges were used in experiments. In some examples the ratio of dry nanofibrillar cellulose in the gel to the cryoprotectants (NFC:PEG:trehalose) is in the range of 5-40:2-6:1 by weight, 9-22:2-5:1 by weight, or 9.5-20:2-5:1 by weight, or 9-22:2-4:1 by weight, or 9.5-20:2-4:1 by weight, for example about 10:5:1 by weight, about 15:5:1 by weight, about 10:3:1 by weight, about 15:3:1 by weight or about 20:3:1 by weight.

The obtained mixture is freeze-dryable, i.e. the freeze-drying process does not have remarkable effect to the physical properties of the dried nanofibrillar gel.

The mixture may be also called as a freeze-dryable hydrogel. One example of such physical property is a release profile of an agent from the hydrogel, such as small or large molecule(s), for example the substances in the cell-free tissue extract, or further therapeutic or cosmetic agent(s). It was found out that different molecules having a variety of molecular weights, including relatively small organic molecules as well as larger proteins, could be released from the hydrogel in a controlled way with similar release profile. The useful molecular weight range is very broad, for example in the tests molecules having a molecular weight in the range of about 170-70 000 g/mol (Daltons) could be released controllably. However, the molecules with high molecular weight did release slower than the molecules with lower molecular weight.

In one example the method comprises further providing one or more therapeutic agent(s) and mixing the agent(s) with the hydrogel, the cell-free tissue extract, the polyethylene glycol and the trehalose, i.e. adding the agent(s) to the mixture. The therapeutic agent(s) may be added simultaneously with the cell-free tissue extract, the polyethylene glycol and/or trehalose, or the therapeutic agents(s) may be added before or afterwards.

In one example the method comprises further providing one or more cosmetic agent(s) and mixing the agent(s) with the hydrogel, the cell-free tissue extract, the polyethylene glycol and the trehalose, *i.e*. adding the agent(s) to the mixture. The cosmetic agent(s) may be added simultaneously with the polyethylene glycol and/or trehalose, or the cosmetic agents(s) may be added before or afterwards. A combination of therapeutic agent(s) and cosmetic agent(s) may be also used.

After the mixture has been obtained, it is freeze-dried to obtain a dried hydrogel comprising nanofibrillar cellulose. Any suitable freeze-drying method may be used. Freeze drying, which may also be called as lyophilisation, is a method which uses rapid cooling to produce thermodynamic instability within a system and cause phase separation. The solvent is then removed by sublimation under vacuum leaving behind voids in the regions it previously occupied. Sublimation refers to transition of a substance directly from the solid to the gas phase without passing through the intermediate liquid phase. Sublimation is an endothermic phase transition that occurs at temperatures and pressures below a substance's triple point in its phase diagram.

In one example the freeze drying comprises first lowering the temperature of the mixture to at least to -30°C, such as at least -40°C, for example to the range of -30--100°C, or to the range of -40--100°C, or even to about -200°C or below, for example when using liquid nitrogen, and after that applying lowered pressure to remove water from the mixture. In general the mixture should be frozen before applying the lowered pressure. In one example the temperature is increased during applying lowered pressure and after applying the lowest pressure, for example the temperature is increased to about -20°C or even to about -10°C. The temperature may be increased before the lowered pressure is applied, or it may be increased during applying the lowered pressure.

In one example the freeze drying is carried out by freezing the mixture with liquid nitrogen. For example a vial containing the mixture is dipped into liquid nitrogen until the mixture is frozen. After this the lowered pressure is applied to the mixture to remove water from the mixture. The lowered pressure may refer to vacuum required to obtain the sublimation of the water. As the sublimation of the water takes place under the triple point, the required vacuum pressure is dependent on the used temperature.

"Drying" as used herein refers in general to dewatering, which terms may be used interchangeably, wherein water is removed from a hydrogel to obtain dried or dewatered hydrogel. In one example the freeze drying is continued until the hydrogel has a desired moisture content or the freeze drying is continued to a minimum moisture content, preferably below 20%, or more preferably below 10%, or even below 5%, for example to a moisture content in the range of 1-20%, 2-20%, or 2-10% (w/w). In one example the freeze drying is continued until the hydrogel has a moisture content in the range of 2-8%, 2-6%, 2-5% or 1-5% (w/w). In general it may be challenging to obtain a moisture content below 2%. After the low moisture content has been obtained, the dried product may be packed into a package in vacuum or in protective gas. This will prevent the dried hydrogel absorbing the ambient moisture, which might rise the moisture content to a range of for example 4-8%, or 5-7% (w/w).

The obtained dried hydrogel may be regelled by adding aqueous liquid, such as water, and suspending the dried product. A regelled or resuspended hydrogel is obtained, which may have the same concentration and water content as before drying. This hydrogel provides characteristics which are substantially equal to the original hydrogel before drying.

Final dried hydrogels, more particularly freeze-dried hydrogels, comprising nanofibrillar cellulose comprising cell-free tissue extract are obtained with the freeze-drying methods of the embodiments disclosed herein.

One embodiment provides a dried hydrogel comprising nanofibrillar cellulose, cell-free adipose tissue extract comprising a mixture of bioactive substances, polyethylene glycol and trehalose, wherein the moisture content of the dried hydrogel is 10% (w/w) or less, or less than 10% (w/w), such as in the range of 1-20% (w/w), or 2-10% (w/w), as discussed in the previous.

In one embodiment the dry content of the cell-free tissue extract in the dried hydrogel is in the range of 0.1-65% (w/w), or in the range of 0.1-50% (w/w), such as in the range of 1-25% (w/w), or 1-20% (w/w), 1-10% (w/w), 1-5% (w/w), or 20-65% (w/w), 10-65% (w/w), 5-65% (w/w), 10-50% (w/w), 5-50% (w/w), 5-25% (w/w), 5-20% (w/w), or 5-15% (w/w). These ranges may also include any possible therapeutic agent(s).

In one embodiment the content of the polyethylene glycol is in the range of 1-20% (w/w) and/or the content of the trehalose is in the range of 0.5-10% (w/w) in the dried hydrogel. In one example the content of the polyethylene glycol is in the range of 5-15% (w/w) and/or the content of the trehalose is in the range of 3-4% (w/w) in the dried hydrogel.

The dried hydrogel may be provided as sheets, blocks or other shapes or forms, in general suitable for the desired medical purpose, and which may be then rewetted before use to obtain a medical gel which is applicable to the target, such as a wound. The dried hydrogel may also be provided as powder or in other crushed form. In such case the method of preparing the product may contain a step of forming the powder, for example by grinding or crushing the freeze-dried product.

The obtained hydrogels, before or after drying, may be used in variety of applications such as those described herein, for example in a method for delivering substances to a subject. The hydrogel may be provided for example as a medical product.

### Medical products

The medical products comprising the tissue extract in the hydrogel may be used in several applications. One specific field is medical applications, wherein the materials are applied on living tissue, such as skin. Any references in the description to therapeutic methods of treatment refer to the compounds and compositions of the present invention for use in a method for treatment of the human (or animal) body by therapy. The materials may be used in medical products, such as patches, dressings, bandages, filters and the like. The medical products may also be therapeutic products, such as therapeutic patches or gels containing medicament. In general the surface of the product comprising nanofibrillar cellulose will be in contact with the skin during the use. A surface of nanofibrillar cellulose may provide advantageous effects when it is in direct contact with the skin, for example it may promote healing of a wound or other damage on a skin, or it may promote delivery of substances from the medical product to the skin.

The term "wound" as used herein refers to any damages, injuries, diseases, disorders or the like on a tissue, such as skin, mucous membrane, or subcutaneous tissue including tendons, including open or closed wounds, wherein the healing of the wound is desired and may be promoted with the product described herein. The wound may be clean, contaminated, infected or colonized, wherein especially in the latter cases a therapeutic agent, such as an antibiotic, may be administered. Examples of open wounds include abrasions, avulsions, incisions, lacerations, puncture wounds and penetration wounds. Examples of closed wounds include hematomas, crush injuries, sewn wounds, grafts and any skin conditions, diseases or disorders. Examples of conditions, diseases or disorders of the skin include acne, infections, vesiculobullous diseases, cold sore, cutaneous candidiasis, cellulitis, dermatitis and eczema, herpes, hives, lupus, papulosquamous, urticaria and erythema, psoriasis, rosacea, radiation-related disorders, pigmentation, mucinoses keratosis, ulcer, atrophy, and necrobiosis, vasculitis, vitiligo, warts, neutrophilic and eosinophilic diseases, congenital, neoplasms and cancer, such as melanomas and tumours of epidermis or dermis, or other diseases or disorders of epidermis and dermis.

A medical product further comprising a therapeutic agent may be provided, wherein the hydrogel comprising the tissue extract in the nanofibrillar cellulose further contain(s) one or more therapeutic agent(s), such as a bioactive agent, an active agent, a medicament or drug. Also the term pharmaceutical agent may be used interchangeably instead of the term therapeutic agent. Such agents are active or effective agents, which are usually present in effective amounts. The therapeutic agent may be provided in the form of a salt, ester, amide, prodrug, conjugate, active metabolite, isomer, fragment, analog, or the like. The agent may produce a systemic or a local effect in the subject. Such an agent may be provided in a predetermined amount, for example in an amount configured to provide a desired dose of the agent during a certain time period, and/or configured to provide a desired effect on the target, such as skin or other tissue. The content of the therapeutic agent in the product may be for example in the range of 0.01-20% (w/w), such as 0.05-10% (w/w). In one example the content of the therapeutic agent in the product is in the range of 0.1-5% (w/w), such as 0.1-3% (w/w), 0.5-3.5% (w/w) or 0.5-5% (w/w). Especially if the therapeutic agent is included, a controlled, sustained or prolonged release of the agent may be provided. In such case the nanofibrillar cellulose may contain a portion of moisture to enable permeability of the agent. The therapeutic agents may be present in water-soluble form, fat-soluble form or in an emulsion, or in another suitable form.

Examples of therapeutic or bioactive agents which may be further included to the medical products described herein include proteins, peptides, carbohydrates, lipids, nucleic acids or fragments thereof, preferably as isolated; antibiotics, pain relievers, such as lidocaine; opioids, such as fentanyl or buprenorphine; nicotine; hormones, such as estrogen, contraceptives or androgens, such as testosterone; nitroglycerin; scopolamine; clonidine; antidepressants, such as selegiline; ADHD medication, such as methylphenidate; vitamins, such as B12 or cyanocobalamin; 5-hydroxytryptophan; Alzheimer's medication, such as rivastigmine; acne medication; antipsoriatics, glucocorticoids such as hydrocortisone; antiandrogens such as bifluranol, cyoctol, cyproterone, delmadinone acetate, flutimide, nilutamide and oxendolone; antiestrogens such as delmadinone acetate, ethamoxytriphetol, tamoxifen and toremifene; antimicrobial agents; anesthetics; analgesics; anti-inflammatory compounds or agents; antihistamines; beta-blockers; growth factors; immunomodulators or medicament for treating diseases or disorders of skin. The tissue extracts alone or together with one or more therapeutic agent(s) may be included for example in medical patches, which may be used on healthy skin or on damaged skin, to provide a prolonged, sustained or extended release of the therapeutic agent from the patch, for example during a period of several hours, for up to 6, 12, 24 or even 48 hours.

"Prolonged release", also called as timed release, sustained release or extended release, refers to a drug, or to a carried impregnated with the drug, that is designed to deliver a dose of a medication over an extended period. The aim is to maintain drug concentration within the therapeutic window for maximum or desirable period of time. The terms are generally used in context of oral dosage forms. In addition to pills, capsules and injectable drug carriers (that often have an additional release function), forms of controlled release medicines include gels, implants and devices and transdermal patches. The definition in European Pharmacopoeia recites: "A prolonged-release dosage form is a modified-release dosage form showing a slower release of the active substance(s) than that of a conventional-release dosage form administered by the same route. Prolonged release is achieved by special formulation design and/or manufacturing method. Equivalent term: extended-release dosage form."

One example provides the medical product further comprising antibiotic agent. Such a product is especially suitable for treating wounds, wherein the wound treating properties are combined with antibiotic properties which prevents infections caused by harmful microbes in the wound. Examples of suitable antibiotics include especially topical antibiotics, such as bacitracin, erythromycin, clindamycin, gentamycin, neomycin, polymyxin, mupirocin, tetracycline, meclocycline, (sodium) sulfacetamide, benzoyl peroxide, and azelaic acid, and combinations thereof. Also other types of antibiotics, such as systemic antibiotics, may be provided, for example penicillins, such as phenoxymethylpenicillin, flucloxacillin and amoxicillin; cephalosporins, such as cefaclor, and cefadroxil; tetracyclines, such as tetracycline, doxycycline and lymecycline; aminoglycosides, such as gentamicin and tobramycin; macrolides, such as erythromycin, azithromycin and clarithromycin; clindamycin; sulphonamides and trimethoprim; metronidazole and tinidazole; quinolones, such as ciprofloxacin, levofloxacin and norfloxacin.

Examples of androgens include boldenone, fluoxymesterone, mestanolone, mesterolone, methandrostenolone, 17-methyltestosterone, 17-alpha-methyltestosterone 3-cyclopentyl enol ether, norethandrolone, normethandrone, Oxandrolone, Oxymesterone, oxymetholone, Prasterone, Stanlolone, Stanozolol, testosterone, testosterone 17-chloral hemiacetal, testosterone 17-beta-cypionate, testosterone enanthate, testosterone nicotinate, testosterone pheynylacetate, testosterone propionate and tiomesterone.

Examples of antibiotics that may be included in the hydrogels include aminoglycosides (e.g., tobramycin, amikacin, gentamicin, kanamycin, netilmicin, tobramycin, streptomycin, azithromycin, clarithromycin, erythromycin, neomycin, erythromycin estolate/ ethylsuccinate, gluceptate/lactobionate/stea rate), beta-lactams such as penicillins (e.g., penicillin G, penicillin V, methicillin, nafcillin, oxacillin, cloxacillin, dicloxacillin, ampicillin, amoxicillin, ticarcillin, carbenicillin, mezlocillin, azlocillin and piperacillin), cephalosporins (e.g., cephalothin, cefazolin, cefaclor, cefamandole, cefoxitin, cefuroxime, cefonicid, cefinetazole, cefotetan, cefprozil,loracarbef, cefetamet, cefoperazone, cefotaxime, ceftizoxime, ceftriaxone, ceftazidime, cefepime, cefixime, cefpodoxime, and cefsulodin), fluoroquinolones (e.g.,ciprofloxacin), carbepenems (e.g., imipenem), tetracyclines (e.g., doxycycline, minocycline, tetracycline), macrolides (e.g., erythromycin and clarithromycin), monobactams (e.g., aztreonam), quinolones (e.g., fleroxacin, nalidixic acid, norfloxacin, ciprofloxacin, ofloxacin, enoxacin, lomefloxacin and cinoxacin), glycopeptides (e.g. vancomycin, teicoplanin), chloramphenicol, clindamycin,trimethoprim, sulfamethoxazole, nitrofurantoin, rifampin and mupirocin, and polymyxins, such as PMB, oxazolidinones, imidazoles (e.g., miconazole, ketoconazole, clotrimazole, econazole, omoconazole, bifonazole, butoconazole, fenticonazole,isoconazole, oxiconazole, sertaconazole, sulconazole and tioconazole), triazoles (e.g.,fluconazole, itraconazole, isavuconazole, ravuconazole, posaconazole, voriconazole,terconazole and albaconazole), thiazoles (e.g., abafungin), and allylamines (e.g.,terbinafine, naftifine and butenafine), echinocandins (e.g., anidulafungin, caspofunginand micafungin). Other antibioties can include polygodial, benzoic acid, ciclopirox,tolnaftate, undecylenic acid, flucytosine or 5-fluorocytosine, griseofulvin, andhaloprogin.

Antibiotics may be also used for treating acne, for example clindamycin, erythromycin, doxycycline, tetracycline etc. Also other agents may be used, such as benzoyl peroxide, salicylic acid, topical retinoid medicines, such as tretinoin, adapalene or tazarotene, azelaic acid, or androgen blockers such as spirolactone. Psoriasis may be treated for example with steroids, such as corticosteroids, moisturizers, calciprotriene, coal tar, vitamin D, retinoids, tazatorene, anthralin, salisylic acid, methotrexate, or cyclosporine. Insect bites or poison ivy exposure may be treated with agents such as hydrocortisone, emu oil, almond oil, ammonia, bisabolol, papain, diphenylhydramine, jewelweed extract or calamine. Some of these or other treatment agents may be also categorized as cosmetic agents.

Examples of antimicrobial agents that may be included in the hydrogels include silver particles, particularly silver nanoparticles, agents or compounds that release silver ions, chlorhexidine gluconate, and polyhexamethylene biguanide.

Examples of anesthetics that may be included in the hydrogels include procaine, benzocaine, chloroprocaine, cocaine, cyclomethycaine, dimethocaine, piperocaine, propoxycaine, procaine, novocaine, proparacaine, tetracaine, lidocaine, articaine, bupivacaine, cinchocaine, etidocaine, levobupivacaine, mepivacaine, prilocaine, ropivacaine, and trimecaine. In some examples, the anesthetic is a combination of lidocaine and prilocaine.

Examples of analgesics that may be included in the hydrogels include opiates and analogues thereof. Exemplary opiates include morphine, codeine, oxycodone, hydrocodone, dihydromorphine, pethidine, buprenorphine, tramadol, fentanyl and venlafaxine.

Examples of anti-inflammatory compounds that may be included in the hydrogels include hydrocortisone, cortisone, dexamethasone, fluocinolone, triamcinolone, medrysone, prednisolone, flurandrenolide, prednisone, halcinonide, methyl prednisolone, prednisone, halcinonide, fludrocortisone, corticosterone, paramethasone, betamethasone, ibuprophen, naproxen, fenoprofen, fenbufen, flurbiprofen, indoprofen, ketoprofen, suprofen, indomethacin, piroxicam, acetosalicylic acid, salicylic acid, diflunisal, methyl salicylate, phenylbutazone, sulindac, mefenamic acid, meclofenamate sodium and tolmetin.

Examples of antihistamines that may be included in the hydrogels include diphenhydramine, dimenhydrinate, perphenazine, triprolidine, pyrilamine, chlorcyclizine, promethazine, carbinoxamine, tripelennamine, brompheniramine, hydroxyzine, cyclizine, meclizine, clorprenaline, terfenadine and chlorpheniramine.

Examples of growth factors that may be included in the hydrogels include vascular endothelial growth factor ("VEGF"), nerve growth factor, such as NGF-beta, plateletderived growth factor (PDGF), fibroblast growth factors, including, for instance, aFGF and bFGF, epidermal growth factor (EGF), keratinocyte growth factor, tumor necrosis factor, transforming growth factors (TGF), including, among others, TGF-alpha and TGF-beta, including TGF-beta-1, TGF-beta-2, TGF-beta-3, TGF-beta-4, or TGF-beta-5, insulin-like growth factors-I and -II (IGF-I and IGF-II), des(1-3)-IGF-I (brain IGF-1), neurotrophin-3 (NT-3) and brain-derived neurotrophic factor (BDNF).

Examples of immunomodulators that may be included in the hydrogels include cyclosporin A, guanylhydrazone, azathioprine, methotrexate, cycphosphamide and tacrolimus.

One example provides a medical product, such as a dressing, a patch or a filter, comprising the hydrogel described herein.

The freeze-dried hydrogel described herein may be provided as an implant, or as any other suitable medical device, for example a delivery device, configured to be introduced or implanted into a body of a subject, such as a patient or any other subject in the need thereof. The implant comprising the freeze-dried medical hydrogel may be introduced within tissue, a body lumen, or other location such that the hydrogel is exposed to bodily fluids or other aqueous environment. The implant may have a surface area in the range of 10-1000 mm², for example in the range of 300-600 mm². The implant may have a suitable size and shape, for example cylindrical shape. In one example the length of a cylindrical implant is in the range of 5-60 mm, and the diameter is in the range of 1.5-5 mm. The implant may be hydrated or wetted prior implanting, or it may be arranged to be hydrated in the body after implanted.

One example provides the hydrogel containing the cell-free tissue extract, especially adipose tissue extract, for use for treating and/or covering skin wounds or other damages. One example provides such a hydrogel for use as a dressing or a patch, or in a dressing or a patch, for treating and/or covering skin wounds or other damages.

One example provides the hydrogel containing the cell-free tissue extract, especially adipose tissue extract, for use for treating and/or covering subcutaneous soft tissue damages, such as subcutaneous soft tissue damages caused by wound, other damage, or surgery.

One example provides the hydrogel containing the cell-free tissue extract, especially adipose tissue extract, for use for treating and/or covering skin wounds covered with a graft, such as a skin graft. One example provides the hydrogel containing the cell-free tissue extract, especially adipose tissue extract, for use as a dressing or a patch, or in a dressing or a patch, for treating and/or covering skin wounds covered with a graft, such as a skin graft.

One example provides the hydrogel containing the cell-free tissue extract, especially adipose tissue extract, for use for administering a further therapeutic agent, more particularly one or more therapeutic agent(s). In one example the hydrogel may be provided as such or for example in a patch. In one example the hydrogel may be provided in injectable form. One or more therapeutic agent(s) may be included, for example impregnated, in the hydrogel as described herein, and the administration to a patient may be for example dermal, transdermal or subcutaneous.

One example provides a cosmetic product, such as a dressing, a mask or a patch, comprising the hydrogel containing the cell-free tissue extract, especially adipose tissue extract,. Such a product may be called also as a cosmetic product. The product may be provided in various shapes, for example a mask may be designed to fit onto face, for example below eye or onto chin, nose or forehead. One example provides the hydrogel for use as a cosmetic product. The product may be used for releasing one or more cosmetic agent(s) to the user, such as to the skin of the user. Such a cosmetic product may comprise one or more cosmetic agent(s). Cosmetic agent(s) may be included, for example impregnated, in the product wherefrom they will be released or delivered. The content of a cosmetic agent in the product may be for example in the range of 0.01-20% (w/w), such as 0.05-10% (w/w). In one example the content of the cosmetic agent in the product is in the range of 0.1-5% (w/w), such as 0.1-3% (w/w), or 0.5-5% (w/w). The cosmetic agents may be present or provided in the product similarly as explained above for therapeutic agents, and vice versa. The cosmetic use may be analogous to medical use described herein, especially the administering of therapeutic agent. Cosmetic agents may be used also for cosmetically treating skin diseases or disorders, such as those mentioned herein. Such cosmetic products may be used for example for treating pimples, acneic skin, brown sports, wrinkles, oily skin, dry skin, aged skin, spider veins, after sun erythemas, black circles etc. Examples of cosmetic patches include skin cleansers, such as pore cleansers, blackhead removers, stretching stripes, short-term patch-like masks, short-term treatment patches and overnight treatment patches.

Examples of cosmetic agents include forms of vitamins and precursors thereof, such as vitamin A; for example retinoids, such as retinaldehyde (retinal), retinoic acid, retinyl palmitate and retinyl retinoate, ascorbic acid, alpha-hydroxy acids such as glycolic acid and lactic acid; glycols; biotechnology products; keratolytics; amino acids; antimicrobials; moisturizers; pigments; antioxidants; plant extracts; cleansing agents or make-up removers; anti-cellulite agents such as caffeine, carnitine, ginkgo biloba and horse-chestnut; conditioners; fragrances such as aromatherapy agents and perfumes; humectants such as urea, hyaluronic acid, lactic acid and glycerine; emollients such as lanolin, triglycerides and fatty acid esters; FR scavengers, singlet oxygen scavengers, superoxide scavengers or hydrogen peroxide scavengers, such as ascorbic acid (vitamin C), glutathione, tocopherol (vitamin E), carotenoids, coenzyme Q10, bilirubin, lipoic acid, uric acid, enzyme mimetic agents, idebenone, polyphenols, selenium, spin traps such as phenyl butyl nitrone (PBN), protein methionine groups, superoxide dismutase, catalase, selenium peroxidases, heme oxygenases etc. or combinations thereof. The cosmetic agents may be present in water-soluble form, fat-soluble form or in an emulsion, or in another suitable form.

The medical or cosmetic hydrogels as described herein may be provided as incorporated or packed in an application device, such as a syringe, an applicator, a pump or a tube containing the desired amount of the hydrogel, such as syringes of the size from 0.5 ml to 200 ml or even more. The device may comprise a mouthpiece or nozzle providing constant flow of the hydrogel in desired thickness and breadth and geometries. In one example the hydrogel is in an injectable form having a concentration and viscosity in such range that enables the injection, for example though a needle or through the syringe mouthpiece or nozzle. A freeze-fried hydrogel may be provided as packed in a sealed package, for example in vacuum package or a package containing protective gas. The hydrogel may be, especially in the freeze-dried form, in a form of an implant, which may be for example a medical capsule, or the like. The hydrogel may be in a form of a sheet or the like which may be applied to a wound or other target after it has been moisturized. These "ready for use" devices may be packed, sterilized and stored, and used when desired. These application devices may be incorporated in ready-to use kits. The moisture content of the freeze-dried hydrogel, or the product containing thereof, such as a product comprising therapeutic agent, may be in the range of 1-10% (w/w), such as in the range of 2-10% (w/w), 2-8% (w/w), 1-5% (w/w), 2-5% (w/w), or 5-7% (w/w). In one example such as package is provided with a separate container containing liquid, such as water, saline solution, buffer solution or the like aqueous liquid in a predetermined amount arranged to rehydrate the gel into a desired moisture content, as described herein. The liquid may be provided for example in a syringe or the like applicator, or a package may contain a separate compartment for the liquid which may be connected to another compartment containing the dried hydrogel, for example by pressing the package and breaking a seal or the like to cause the liquid and the dried gel to be in contact with each other.

One example provides a method for cosmetically treating skin, the method comprising applying the medical product or the cosmetic product described herein onto skin.

One example provides the medical product or the cosmetic product described herein packed in a separate packing. Separate packings may be provided as a series of packings. Usually such packed products are provided as sterilized.

One example provides a kit comprising the medical product or the cosmetic product described herein, for example a packed product, wherein the kit may contain one or more of the packed products. The kit may also contain other materials or equipment, such as a container containing water or other aqueous solution, such as saline solution or the like, for pretreating the product(s) prior to use, for example a predetermined amount of water or other aqueous solution for obtaining a desired moisture content of the re-gelled product.

One example provides a method for delivering or administering substances to a subject, the method comprising providing the medical hydrogel as described in the embodiments containing the cell-free tissue extract and optionally one or more other substance(s), such as therapeutic or cosmetic substance(s) or agent(s), and applying the hydrogel onto skin of the subject. The subject may be a patient or any other subject in need of the substance(s), such as human or animal. By applying the hydrogel onto the skin, the substance(s) will be delivered transdermally, preferably by controlled and/or prolonged release rate.

One example provides a method for delivering substances to a subject, the method comprising providing the medical hydrogel, as described in the embodiments, containing the cell-free tissue extract and optionally one or more other substance(s), such as therapeutic or cosmetic substance(s) or agent(s), and injecting the hydrogel to the subject. The injection, or more precisely the administration route, may be subcutaneous or intramuscular.

One example provides a method for treating skin wounds or other damages or injuries, the method comprising applying the medical product described herein onto the wound, damage, or injury. One specific example provides a method for treating skin wounds covered with a graft, such as a skin graft, for example a mesh graft or a full thickness graft, the method comprising applying the medical product described herein onto the graft.

Grafting refers to a surgical procedure to move tissue from one site to another on the body, or from another person, without bringing its own blood supply with it. Instead, a new blood supply grows in after it is placed. Autografts and isografts are usually not considered as foreign and, therefore, do not elicit rejection. Allografts and xenografts are recognized as foreign by the recipient and are rejected.

Skin grafting is often used to treat skin loss due to a wound, burn, infection, or surgery. In the case of damaged skin, it is removed, and new skin is grafted in its place. Skin grafting can reduce the course of treatment and hospitalization needed, and can also improve function and appearance. There are two types of skin grafts: Split-thickness skin grafts (epidermis + part of the dermis) and full-thickness skin grafts (epidermis + entire thickness of the dermis).

A mesh graft is a full- or partial-thickness sheet of skin that has been fenestrated to allow drainage and expansion. Mesh grafts are useful in many locations on the body because they conform to uneven surfaces. They can be placed in locations that have excessive motion because they can be sutured to the underlying wound bed. Additionally, their fenestrations provide outlets for fluid that may accumulate beneath the graft, which helps reduce tension and the risk of infection and improve vascularization of the graft.

Before applying the medical product onto skin the product may be pretreated *i.e.* moisture or wetted, in general with an aqueous solution. The moisturizing or wetting may be carried out for example by using water or regular physiological saline solution, which is usually a solution of 0.90% w/w of NaCI, having an osmolality of about 308 mOsm/l. Other types of aqueous solutions may also be used, such as saline solutions with different concentrations.

Moisturizing or wetting the material enhances contact with the skin and the moldability of a sheet of material.

### Examples

In the tests it was discovered and shown that TEMPO oxidized NFC hydrogel can be successfully freeze dried and redispersed into a hydrogel form. The used cryoprotectants, PEG6000 and trehalose, together significantly improved the storage and loss modulus of the rehydrated and redispersed NFC hydrogels after freeze drying as opposed to formulations without these cryoprotectants. The viscosity of the freeze dried NFC hydrogels with excipients was also preserved upon rehydration.

Adipose tissue extract was mixed with about 3.2% and 6% (w/w) nanofibrillar cellulose gels and freeze-dried as described herein. Redispersable dried products were obtained exhibiting the bioactivities present in the original adipose tissue extract.

These finding indicate that cryoprotectants aided in preserving the structure of the NFC fibers during the freeze drying process. The production of NFC aerogels therefore did not significantly alter rheological properties. Furthermore, the bioactive substance release profiles of the bioactive compounds were similar before and after freeze drying. This is a highly desirable feature for tissue extract formulations as the shelf-life of the product can be increased by the dry state of aerogel for otherwise hydrolysis sensitive tissue extracts. The aerogels can be easily rehydrated and administered upon need.

## Claims

1. A method for drying cell-free adipose tissue extract in a hydrogel comprising nanofibrillar cellulose, the method comprising
- providing a hydrogel comprising nanofibrillar cellulose,
- providing cell-free adipose tissue extract comprising a mixture of bioactive substances,
- providing polyethylene glycol,
- providing trehalose,
- mixing the hydrogel, the cell-free adipose tissue extract, the polyethylene glycol and the trehalose to obtain a mixture, and
- freeze drying the mixture to obtain a dried cell-free adipose tissue extract in a dried hydrogel comprising nanofibrillar cellulose.

2. The method of claim 1, wherein the cell-free adipose tissue extract is obtained from tissue obtained directly from a body.

3. The method of any of the preceding claims, wherein the cell-free adipose tissue extract is cell-free human adipose tissue extract.

4. The method of claim 3, wherein the cell-free human adipose tissue extract contains at least VEGF, FGF-2, and IGF-1.

5. The method of any of the preceding claims, wherein the dry content of the cell-free adipose tissue extract in the mixture is in the range of 0.01-10% (w/w), such as in the range of 0.1-5% (w/w) calculated from the total mass of the mixture.

6. The method of any of the preceding claims, wherein the mixture contains 0.1-2% (w/w) of polyethylene glycol and/or 0.05-1.0% (w/w) of trehalose calculated from the total mass of the mixture.

7. The method of any of the preceding claims, wherein the nanofibrillar cellulose, when dispersed in water, provides a Brookfield viscosity of at least 2000 mPa·s, such as at least 3000 mPa·s, for example at least 10000 mPa·s, such as in the range of 2000-20000 mPa·s, measured at 20°C±1°C, at a consistency of 0.8% (w/w) and at 10 rpm.

8. The method of any of the preceding claims, wherein the concentration of the nanofibrillar cellulose in the hydrogel before the freeze-drying is in the range of 0.5-10%, such as 2-8%, for example 3-7%.

9. The method of any of the preceding claims, wherein the nanofibrillar cellulose is selected from anionically modified nanofibrillar cellulose, cationically modified nanofibrillar cellulose and unmodified nanofibrillar cellulose, and TEMPO oxidized nanofibrillar cellulose.

10. The method of any of the preceding claims, wherein the freeze drying is continued until the dried hydrogel has a moisture content 10% or less, such as in the range of 2-10% (w/w), for example 2-8% (w/w) or 2-5% (w/w).

11. A dried hydrogel comprising nanofibrillar cellulose, cell-free adipose tissue extract comprising a mixture of bioactive substances, polyethylene glycol and trehalose, wherein the moisture content of the dried hydrogel is 10% (w/w) or less, such as in the range of 2-10% (w/w), for example 2-8% (w/w) or 2-5% (w/w).

12. The dried hydrogel comprising nanofibrillar cellulose of claim 11, wherein the dry content of the cell-free adipose tissue extract in the dried hydrogel is in the range of 0.1-65% (w/w), such as in the range of 0.1-50% (w/w), for example in the range of 1-25% (w/w).

13. The dried hydrogel comprising nanofibrillar cellulose of claim 11 or 12, wherein the content of the polyethylene glycol is in the range of 1-20% (w/w) and/or the content of the trehalose is in the range of 0.5-10% (w/w) in the dried hydrogel.

14. The dried hydrogel comprising nanofibrillar cellulose of any of the claims 11-13, wherein the cell-free adipose tissue extract is obtained from tissue obtained directly from a body.

15. The dried hydrogel comprising nanofibrillar cellulose of any of the claims 11-14, wherein the cell-free adipose tissue extract is cell-free human adipose tissue extract.

16. The dried hydrogel comprising nanofibrillar cellulose of claim 15, wherein the cell-free human adipose tissue extract contains at least VEGF, FGF-2, and IGF-1.

17. The dried hydrogel comprising nanofibrillar cellulose of any of the claims 11-16, wherein the nanofibrillar cellulose, when dispersed in water, provides a Brookfield viscosity of at least 2000 mPa·s, such as at least 3000 mPa·s, for example at least 10000 mPa·s, such as in the range of 2000-20000 mPa·s, measured at 20°C±1°C, at a consistency of 0.8% (w/w) and at 10 rpm.

18. The dried hydrogel comprising nanofibrillar cellulose of any of the claims 11-17, wherein the nanofibrillar cellulose is selected from anionically modified nanofibrillar cellulose, cationically modified nanofibrillar cellulose and unmodified nanofibrillar cellulose, and TEMPO oxidized nanofibrillar cellulose.

19. The dried hydrogel comprising nanofibrillar cellulose of any of the claims 11-18 obtained with the method of any of the claims 1-10.

## Patentansprüche

1. Verfahren zum Trocknen von zellfreiem Fettgewebeextrakt in einem Hydrogel, welches nanofibrilläre Cellulose umfasst, wobei das Verfahren Folgendes umfasst
- Bereitstellen eines Hydrogels, das nanofibrilläre Cellulose umfasst,
- Bereitstellen von zellfreiem Fettgewebeextrakt, der eine Mischung aus bioaktiven Substanzen umfasst,
- Bereitstellen von Polyethylenglykol,
- Bereitstellen von Trehalose,
- Vermischen des Hydrogels, des zellfreien Fettgewebeextrakts, des Polyethylenglykols und der Trehalose, um eine Mischung zu erhalten, und
- Gefriertrocknen der Mischung, um einen getrockneten zellfreien Fettgewebeextrakt in einem getrockneten Hydrogel zu erhalten, welches nanofibrilläre Cellulose umfasst.

2. Verfahren nach Anspruch 1, wobei der zellfreie Fettgewebeextrakt ausgehend von Gewebe erhalten wird, das unmittelbar einem Körper entnommen wurde.

3. Verfahren nach beliebigen der vorhergehenden Ansprüche, wobei es sich bei dem zellfreien Fettgewebeextrakt um einen zellfreien Extrakt von menschlichem Fettgewebe handelt.

4. Verfahren nach Anspruch 3, wobei der zellfreie Extrakt menschlichen Fettgewebes mindestens VEGF, FGF-2 und IGF-1 enthält.

5. Verfahren nach beliebigen der vorhergehenden Ansprüche, wobei der Trockensubstanzgehalt des zellfreien Fettgewebeextrakts in der Mischung im Bereich 0,01 bis 10 % (w/w) liegt, wie etwa im Bereich von 0,1 bis 5 % (w/w), berechnet ausgehend von der Gesamtmasse der Mischung.

6. Verfahren nach beliebigen der vorhergehenden Ansprüche, wobei die Mischung 0,1 bis 2 % (w/w) an Polyethylenglykol und/oder 0,05 bis 1,0 % (w/w) an Trehalose enthält, berechnet ausgehend von der Gesamtmasse der Mischung.

7. Verfahren nach beliebigen der vorhergehenden Ansprüche, wobei die nanofibrilläre Cellulose, wenn sie in Wasser dispergiert wird, eine Brookfield-Viskosität von mindestens 2.000 mPa.s bereitstellt, wie etwa von mindestens 3.000 mPa.s, beispielsweise von mindestens 10.000 mPa.s, wie etwa im Bereich von 2.000 bis 20.000 mPa.s, gemessen bei 20 °C ± 1 °C, bei einer Konsistenz von 0,8 % (w/w) und bei 10 U/min.

8. Verfahren nach beliebigen der vorhergehenden Ansprüche, wobei die Konzentration der nanofibrillären Cellulose in dem Hydrogel vor dem Gefriertrocknen im Bereich von 0,5 bis 10 %, wie etwa 2 bis 8 %, beispielsweise 3 bis 7 %, liegt.

9. Verfahren nach beliebigen der vorhergehenden Ansprüche, wobei die nanofibrilläre Cellulose aus anionisch modifizierter nanofibrillärer Cellulose, kationisch modifizierter nanofibrillärer Cellulose und unmodifizierter nanofibrillärer Cellulose sowie TEMPO-oxidierter nanofibrillärer Cellulose ausgewählt ist.

10. Verfahren nach beliebigen der vorhergehenden Ansprüche, wobei das Gefriertrocknen fortgesetzt wird, bis das getrocknete Hydrogel einen Feuchtigkeitsgehalt von 10 % oder weniger hat, wie etwa im Bereich von 2 bis 10 % (w/w), zum Beispiel 2 bis 8 % (w/w) oder 2 bis 5 % (w/w).

11. Getrocknetes Hydrogel, das nanofibrilläre Cellulose, zellfreien Fettgewebeextrakt, welcher eine Mischung aus bioaktiven Substanzen umfasst, Polyethylenglykol und Trehalose umfasst, wobei der Feuchtigkeitsgehalt des getrockneten Hydrogels 10 % (w/w) oder weniger beträgt, wobei er etwa im Bereich von 2 bis 10 % (w/w), zum Beispiel 2 bis 8 % (w/w) oder 2 bis 5 % (w/w), liegt.

12. Getrocknetes Hydrogel, das nanofibrilläre Cellulose umfasst, nach Anspruch 11, wobei der Trockensubstanzgehalt des zellfreien Fettgewebeextrakts in dem getrockneten Hydrogel im Bereich von 0,1 bis 65 % (w/w), wie etwa im Bereich von 0,1 bis 50 % (w/w), beispielsweise im Bereich von 1 bis 25 % (w/w), liegt.

13. Getrocknetes Hydrogel, das nanofibrilläre Cellulose umfasst, nach Anspruch 11 oder 12, wobei in dem getrockneten Hydrogel der Gehalt des Polyethylenglykols im Bereich von 1 bis 20 % (w/w) und/oder der Gehalt der Trehalose im Bereich von 0,5 bis 10 % (w/w) liegt.

14. Getrocknetes Hydrogel, das nanofibrilläre Cellulose umfasst, nach beliebigen der Ansprüche 11 bis 13, wobei der zellfreie Fettgewebeextrakt ausgehend von Gewebe erhalten wird, das unmittelbar einem Körper entnommen wurde.

15. Getrocknetes Hydrogel, das nanofibrilläre Cellulose umfasst, nach beliebigen der Ansprüche 11 bis 14, wobei es sich bei dem zellfreien Fettgewebeextrakt um einen zellfreien Extrakt von menschlichem Fettgewebe handelt.

16. Getrocknetes Hydrogel, das nanofibrilläre Cellulose umfasst, nach Anspruch 15, wobei der zellfreie Extrakt menschlichen Fettgewebes mindestens VEGF, FGF-2 und IGF-1 enthält.

17. Getrocknetes Hydrogel, das nanofibrilläre Cellulose umfasst, nach beliebigen der Ansprüche 11 bis 16, wobei die nanofibrilläre Cellulose, wenn sie in Wasser dispergiert wird, eine Brookfield-Viskosität von mindestens 2.000 mPa.s bereitstellt, wie etwa von mindestens 3.000 mPa.s, beispielsweise von mindestens 10.000 mPa.s, wie etwa im Bereich von 2.000 bis 20.000 mPa.s, gemessen bei 20 °C ± 1 °C, bei einer Konsistenz von 0,8 % (w/w) und bei 10 U/min.

18. Getrocknetes Hydrogel, das nanofibrilläre Cellulose umfasst, nach beliebigen der Ansprüche 11 bis 17, wobei die nanofibrilläre Cellulose aus anionisch modifizierter nanofibrillärer Cellulose, kationisch modifizierter nanofibrillärer Cellulose und unmodifizierter nanofibrillärer Cellulose sowie TEMPO-oxidierter nanofibrillärer Cellulose ausgewählt ist.

19. Getrocknetes Hydrogel, das nanofibrilläre Cellulose umfasst, nach beliebigen der Ansprüche 11 bis 18, wobei es mittels des Verfahrens nach beliebigen der Ansprüche 1 bis 10 erhalten wurde.

## Revendications

1. Procédé de séchage d'extrait de tissu adipeux acellulaire dans un hydrogel comprenant la cellulose nanofibrillaire, le procédé comprenant
- la fourniture d'un hydrogel comprenant la cellulose nanofibrillaire,
- la fourniture de l'extrait de tissu adipeux acellulaire comprenant un mélange de substances bioactives,
- la fourniture de polyéthylène glycol,
- la fourniture de tréhalose,
- le mélange de l'hydrogel, de l'extrait de tissu adipeux acellulaire, du polyéthylène glycol et du tréhalose pour obtenir un mélange, et
- la lyophilisation du mélange pour obtenir un extrait de tissu adipeux acellulaire séché dans un hydrogel séché comprenant la cellulose nanofibrillaire.

2. Procédé selon la revendication 1, dans lequel l'extrait de tissu adipeux acellulaire est obtenu à partir de tissu obtenu directement d'un corps.

3. Procédé selon l'une quelconque des revendications précédentes, dans lequel l'extrait de tissu adipeux acellulaire est un extrait de tissu adipeux humain acellulaire.

4. Procédé selon la revendication 3, dans lequel l'extrait de tissu adipeux humain acellulaire contient au moins du VEGF, du FGF-2, et de l'IGF-1.

5. Procédé selon l'une quelconque des revendications précédentes, dans lequel la teneur sèche de l'extrait de tissu adipeux acellulaire dans le mélange se situe dans la plage de 0,01 à 10 % (en pds/pds), telle que dans la plage de 0,1 à 5 % (en pds/pds) calculée à partir de la masse totale du mélange.

6. Procédé selon l'une quelconque des revendications précédentes, dans lequel le mélange contient de 0,1 à 2 % (en pds/pds) de polyéthylène glycol et/ou de 0,05 à 1,0 % (en pds/pds) de tréhalose calculé à partir de la masse totale du mélange.

7. Procédé selon l'une quelconque des revendications précédentes, dans lequel la cellulose nanofibrillaire, lorsqu'elle est dispersée dans l'eau, fournit une viscosité de Brookfield d'au moins 2 000 mPa•s, telle que d'au moins 3 000 mPa•s, par exemple d'au moins 10 000 mPa•s, telle que dans la plage de 2 000 à 20 000 mPa•s, mesurée à 20°C ± 1°C, à une consistance de 0,8 % (en pds/pds) et à 10 tr/min.

8. Procédé selon l'une quelconque des revendications précédentes, dans lequel la concentration de la cellulose nanofibrillaire dans l'hydrogel avant la lyophilisation se situe dans la plage de 0,5 à 10 %, telle que de 2 à 8 %, par exemple de 3 à 7 %.

9. Procédé selon l'une quelconque des revendications précédentes, dans lequel la cellulose nanofibrillaire est sélectionnée parmi la cellulose nanofibrillaire anioniquement modifiée, la cellulose nanofibrillaire cationiquement modifiée et la cellulose nanofibrillaire non modifiée, et la cellulose nanofibrillaire oxydée TEMPO.

10. Procédé selon l'une quelconque des revendications précédentes, dans lequel la lyophilisation se poursuit jusqu'à ce que l'hydrogel séché présente une teneur en humidité de 10 % ou moins, telle que dans la plage de 2 à 10 % (en pds/pds), par exemple de 2 à 8 % (en pds/pds) ou de 2 à 5 % (en pds/pds).

11. Hydrogel séché comprenant la cellulose nanofibrillaire, l'extrait de tissu adipeux acellulaire comprenant un mélange de substances bioactives, de polyéthylène glycol et de tréhalose, où la teneur en humidité de l'hydrogel séché est de 10 % (en pds/pds) ou moins, telle que dans la plage de 2 à 10 % (en pds/pds), par exemple de 2 à 8 % (en pds/pds) ou de 2 à 5 % (en pds/pds).

12. Hydrogel séché comprenant la cellulose nanofibrillaire selon la revendication 11, dans lequel la teneur sèche de l'extrait de tissu adipeux acellulaire dans l'hydrogel sec se situe dans la plage de 0,1 à 65 % (en pds/pds), telle que dans la plage de 0,1 à 50 % (en pds/pds), par exemple dans la plage de 1 à 25 % (en pds/pds).

13. Hydrogel séché comprenant la cellulose nanofibrillaire selon la revendication 11 ou 12, dans lequel la teneur du polyéthylène glycol se situe dans la plage de 1 à 20 % (en pds/pds) et/ou la teneur du tréhalose se situe dans la plage de 0,5 à 10 % (en pds/pds) dans l'hydrogel sec.

14. Hydrogel séché comprenant la cellulose nanofibrillaire selon l'une quelconque des revendications 11 à 13, dans lequel l'extrait de tissu adipeux acellulaire est obtenu à partir de tissu obtenu directement d'un corps.

15. Hydrogel séché comprenant la cellulose nanofibrillaire selon l'une quelconque des revendications 11 à 14,
dans lequel l'extrait de tissu adipeux acellulaire est de l'extrait de tissu adipeux humain acellulaire.

16. Hydrogel séché comprenant la cellulose nanofibrillaire selon la revendication 15,
dans lequel l'extrait de tissu adipeux humain acellulaire contient au moins du VEGF, du FGF-2, et de l'IGF-1.

17. Hydrogel séché comprenant la cellulose nanofibrillaire selon l'une quelconque des revendications 11 à 16, dans lequel la cellulose nanofibrillaire, lorsqu'elle est dispersée dans l'eau, fournit une viscosité de Brookfield d'au moins 2 000 mPa•s, telle que d'au moins 3 000 mPa•s, par exemple d'au moins 10 000 mPa•s, telle que dans la plage de 2 000 à 20 000 mPa•s, mesurée à 20°C ± 1°C, à une consistance de 0,8 % (en pds/pds) et à 10 tr/min.

18. Hydrogel séché comprenant la cellulose nanofibrillaire selon l'une quelconque des revendications 11 à 17, dans lequel la cellulose nanofibrillaire est sélectionnée parmi la cellulose nanofibrillaire anioniquement modifiée, la cellulose nanofibrillaire cationiquement modifiée et la cellulose nanofibrillaire non modifiée, et la cellulose nanofibrillaire oxydée TEMPO.

19. Hydrogel séché comprenant la cellulose nanofibrillaire selon l'une quelconque des revendications 11 à 18 obtenu selon le procédé selon l'une quelconque des revendications 1 à 10.
